# EUROPEAN PATENT SPECIFICATION

(11) **EP 2 894 980 B1**
(45) Date of publication and mention of the grant of the patent: **23.12.2020**
(21) Application number: 13792094.8
(22) Date of filing: 12.09.2013
(51) Int. Cl.: A01N 37/46, A01N 25/26, A01N 25/34, A61L 27/34, A61K 38/10, A61K 38/17, A61L 27/54, A01P 1/00

(54) **ANTIMICROBIAL COATING COMPOSITIONS**
ANTIMIKROBIELLE BESCHICHTUNGSZUSAMMENSETZUNGEN
COMPOSITIONS DE REVÊTEMENT ANTIMICROBIEN

(30) Priority: 12.09.2012 PT 12106532 U
(43) Date of publication of application: 22.07.2015
(73) Proprietor: Biocant - Centro de Inovação em Biotecnologia, Cantanhede 3060-197 (PT)
(72) Inventor: DA SILVA FERREIRA, Lino, 3030-036 Coimbra (PT); RAI, Akhilesh, 3000-135 Coimbra (PT)
(74) Representative: Silvestre de Almeida Ferreira, Luís Humberto
(86) International application number: PCT/IB2013/058502
(87) International publication number: WO 2014/041508

(56) References cited:
- WO-A2-2007/095393
- WO-A2-2010/014940
- US-A1- 2008 292 671
- S. RUDEN ET AL: "Synergistic Interaction between Silver Nanoparticles and Membrane-Permeabilizing Antimicrobial Peptides", ANTIMICROBIAL AGENTS AND CHEMOTHERAPY, vol. 53, no. 8, 1 August 2009 (2009-08-01) , pages 3538-3540, XP055103999, ISSN: 0066-4804, DOI: 10.1128/AAC.01106-08
- O. YU. GOLUBEVA ET AL: "Synthesis and study of antimicrobial activity of bioconjugates of silver nanoparticles and endogenous antibiotics", GLASS PHYSICS AND CHEMISTRY, vol. 37, no. 1, 1 February 2011 (2011-02-01), pages 78-84, XP055103974, ISSN: 1087-6596, DOI: 10.1134/S1087659611010056
- MARCELO J KOGAN ET AL: "Peptides and metallic nanoparticles for biomedical applications", NANOMEDICINE, vol. 2, no. 3, 1 June 2007 (2007-06-01), pages 287-306, XP055104016, ISSN: 1743-5889, DOI: 10.2217/17435889.2.3.287
- NGOC THI THANH TRAN ET AL: "Direct Synthesis of Rev Peptide-Conjugated Gold Nanoparticles and Their Application in Cancer Therapeutics", BIOCONJUGATE CHEMISTRY, vol. 22, no. 7, 20 July 2011 (2011-07-20), pages 1394-1401, XP055104069, ISSN: 1043-1802, DOI: 10.1021/bc2001215
- SOONHYANG PARK ET AL: "Antimicrobial activity and cellular toxicity of nanoparticlepolymyxin B conjugates;Antimicrobial activity and cellular toxicity of nanoparticlepolymyxin B conjugates", NANOTECHNOLOGY, IOP, BRISTOL, GB, vol. 22, no. 18, 17 March 2011 (2011-03-17), page 185101, XP020190267, ISSN: 0957-4484, DOI: 10.1088/0957-4484/22/18/185101

## Description

### Technical field

The disclosed subject-matter relates to an antimicrobial coating composition for coating articles comprising an antimicrobial peptide; a nanoparticle bound to said antimicrobial peptide; wherein the composition is suitable to be bonded to the surface of the article. The said composition could be used as coating composition of medical devices and laboratory surfaces.

### Background

Metal nanoparticles have been extensively investigated for biosensor, drug delivery, antimicrobial materials and other biomedical applications due to their distinct surface plasmon resonance (SPR) properties, stability, biocompatibility, ease of synthesis and surface functionalization¹. There are several reports available in literature about the preparation of antimicrobial metal nanoparticles with broad antimicrobial spectrum^{2,3}. These methods either involve multiple steps including synthesis of nanoparticles, surface functionalization and capping of antimicrobial materials or one pot method to synthesize nanoparticles with antimicrobial properties³. However, there is possibility that bacteria can adopt resistance against these nanomaterials due to their widespread and indiscriminate exposure over time, instigating worldwide public health concerns. Numerous studies have documented about the resistance to nanosilver and nanoalumina to different strains of bacteria through multiple mechanisms⁴. In fact, this resistance can also impact the resistance of bacteria against other kinds of antimicrobial agents⁴. Additionally, toxicity of these nanoparticles to human cells depends on size, shape, functionality and dosage and no metabolic and immunological responses induced by these particles are studied in details⁵.

There is urgent need to develop antimicrobial nanomaterials with properties of not triggering resistance to bacteria and toxicity to human cells. Antimicrobial peptides (AMPs), described later, are a promising class of molecules to meet these criteria due to their mode of action and they represent new generation of antibiotics. Recently, self-assembled AMP nanoparticles, AMPs loaded on carbohydrate nanoparticles and AMP in combination with silver nanoparticles have been used as potent antimicrobial agents⁶. The antimicrobial activity of AMPs may be impaired by proteolytic degradation and therefore various analogues of AMPs with varying chain lengths have been synthesized to prevent the degradation⁷. On the other hand, the capping of peptides on the nanoparticle surface is alternative approach to prevent them from proteolytic degradation^{4f,8}. Peptides with different biological properties have been used to conjugate the gold nanoparticles during the synthesis step for their potential biological applications⁹. Moreover, engineered peptides have been synthesized using selection techniques such as phage, cell surface and yeast display to synthesize metal nanoparticles¹⁰. These peptides not only bind specifically and strongly to a range of different metal, semiconductor, oxide and biomineral surfaces but also precisely control the shape and the size of inorganic nanoparticles mineralized from the atom up¹⁰. However, a number of questions arise about the mechanism of how peptides control morphology and the size of nanoparticles. If the synthesis mechanism will be better understood, biologically significant peptides may be used to synthesize advanced materials for several applications in biocatalysis, imaging, health and therapeutics. To the best of our knowledge, no efforts have been made to synthesize antimicrobial and biocompatible gold nanoparticles using AMPs.

Furthermore, implants and biomedical devices associated infections are very common in patients, which pose serious challenges to medical and healthcare industries. Implant removal and replacement with a new implant are often practiced, which is indeed an expansive procedure. The biofilms formed on the surface of implant protects microorganism from conventional administered antibiotics and immune response from human body. Beyond that, the exposure of antibiotics for longer periods can promote the development of antibiotic resistance phenotypes, which lead to devastating effects to control the infection in absence of valid medical treatments. Various covalent and noncovalent coatings have been developed using antibiotics *(*Rai, A.; Prabhune, A.; Perry, C. C. J. Mater. Chem. 2010, 20, 6789*. b)* Shukla, A.; Avadhany, S. N.; Fang, J. C. Hammond, P. T. Small 2010, 21, 2392*. c)* Hanna, H.; Benjamin, R.; Chatzinikolaou, I.; Alakech, B.; Richardson, D.; Masfield, P.; Dvorak, T.; Munsell, M.F.; Darouiche, R.; Kantarjian, H.; Raad, I. J. Clin. Oncol. 2004, 22, 3263*. d)* Aumsuwan, N.; McConnell, M.S. Urban, M. W. Biomacromol. 2009, 10, 623*),* quaternary ammonium salts *(*Lee, S. B.; Koepsel, R. R.; Morley, S. W.; Matyjaszewski, K.; Sun, Y.; Russell. A. J. Biomacromol. 2004, 5, 877*. b)* Murata, H.; Koepsel. R. R.; Matyjaszewski, K.; Russell, A. J. Biomater. 2007, 28, 4870*),* phenol derivatives *(*Chung, D.; Papadakis, S. E.; Yam, K. L. Int. J. Food Sci. Technol. 2003, 38, 165*),* titanium oxide *(*Fu, G.; Vary, P. S.; Lin, C-T. J. Phys. Chem. B 2005, 109, 8889*. b)* Matsunaga, T.; Tomoda, R.; Nakajima, T.; Nakamura, N.; Komine, T. Appl. Environ. Microbiol. 1988, 54. 1330*)* and heavy metals such as silver *(*Silver, S. Fems Microbiol. Rev. 2003, 27, 341*. b)* Atiyeh, B. S.; Costagliola, M.; Hayek, S. N.; Dibo, S. A. Burns 2007, 33, 139*. c)* Dowling, D. P.; Donnelly, K; McConnell, M. L.; Eloy, R.; Arnaud, M. N. Thin Solid Films 2001, 398, 602*)* and tin derivatives *(*Omae, I. Appl. Organomet. Chem. 2003, 17, 81*)* to combat microbial infection however, there is limited success with these coatings in order to achieve potent antimicrobial surfaces with less cytotoxicity effect. Another drawback with these technologies is that probably microorganisms would develop resistance against these coatings after frequent usages *(*Page, K; Wilson, M.; Parkin, I. P. J. Mater. Chem. 2009, 19, 3819*).* Thus, there is of increasing interest to develop robust coatings for biomedical implants with broad spectrum antimicrobial activity, which would not only kill microorganisms and prevent the growth of biofilms but would also have less effect on the development of bacterial resistance against coatings.

As said before, antimicrobial peptides (AMPs), non-classical drugs, are alternative to other antimicrobial drugs due to their broad-spectrum antimicrobial activities and ability to modulate immune response and no toxicity to host cells *((a)* Onaizi, S. A.; Leong, S. S. J. Biotechnol. Adv. 2011, 29, 67*. b)* Costa, F. Carvalho, I. F. Montelaro, R. C. Gomes, P.; Martins, C. L. Acta Biomaterialia 2011, 7, 1431*. c)* Andreu, D.; Rivas, L. Biopolym. 1998, 47, 4 *and* Hilpert, K; Elliott, M.; Jenssen, H.; Kindrachuk, J.; Fjell, C. D.; Korner, J.; Winkler, D. F. H.; Weaver, L. L.; Henklein, P.; Ulrich, A. S.; Chiang, S. H. Y.; Farmer, S. W.; Pante, N.; Volkmer, R.; Hancock, R. E. W. Chem. Biol. 2009, 16, 58*)* Reddy, K. V.; Yedery, R. D.; Aranha, C. Int. J. Antimicrob. Agents 2004, 24, 536*.).* AMPs are short (15-50 amino acids), highly cationic in nature with a large variation in net positive charge and have tendency to adopt amphipathic structures *((a)* Onaizi, S. A.; Leong, S. S. J. Biotechnol. Adv. 2011, 29, 67*. b)* Costa, F. Carvalho, I. F. Montelaro, R. C. Gomes, P.; Martins, C. L. Acta Biomaterialia 2011, 7, 1431*. c)* Andreu, D.; Rivas, L. Biopolym. 1998, 47, 4 *and* Hilpert, K.; Elliott, M.; Jenssen, H.; Kindrachuk, J.; Fjell, C. D.; Korner, J.; Winkler, D. F. H.; Weaver, L. L.; Henklein, P.; Ulrich, A. S.; Chiang, S. H. Y.; Farmer, S. W.; Pante, N.; Volkmer, R.; Hancock, R. E. W. Chem. Biol. 2009, 16, 58 *and* Reddy, K. V.; Yedery, R. D.; Aranha, C. Int. J. Antimicrob. Agents 2004, 24, 536*;and a)* Perron, G. G.; Zasloff, M.; Bell, G. Proc. Biol. Sci. 2006, 273, 251*. b)* Van't Hof, W.; Veerman, E. C.; Helmerhost, E. J.; Amerongen, A. V. Biol. Chem. 2001, 382, 597*).* In recent year, many strategies such as physical (layer by layer assembly) *((a)* Etienne, O.; Gasnier, C.; Taddei, C.; Voegel, J-C.; Aunis, D.; Schaaf, P. Metz-Boutigue, M-H.; Bolcato-Bellemin, A-L.; Egles, C. Biomater. 2005, 26, 6704*. b)* Etienne, O.; Picart, C.; Taddei, C.; Haikel, Y.; Dimarcq, J. L.; Schaaf, P.; Voegel, J. C.; Ogier, J. A.; Egles, C. Antimicrob. Agents Chemother. 2004, 48, 3662*) and covalent immobilization methods ((a)* Bagheri, M.; Beyermann, M.; Dathe, M. A. Antimicrob. Agents Chemotherap. 2009, 53, 1132*. b)* Haynie, S.L.; Crum, G. A.; Doele, B. A. Antimicrob. Agents Chemotherap. 1995, 39, 301*. c)* Ferreira, L.; Zumbuehl, A J. Mater. Chem. 2009, 19, 7796*)* have been reported in literature for coatings of AMPs on different surfaces with maintenance of their activity. Effects of different parameters including type, length and flexibility of spacers and orientation and concentration of AMPs on the activity of immobilized peptides have been studied to develop efficient, safe and long-lasting antimicrobial implants and devices *((a)* Onaizi, S. A.; Leong, S. S. J. Biotechnol. Adv. 2011, 29, 67*. b)* Costa, F. Carvalho, I. F. Montelaro, R. C. Gomes, P.; Martins, C. L. Acta Biomaterialia 2011, 7, 1431*. c)* Andreu, D.; Rivas, L. Biopolym. 1998, 47, 4 *and* Hilpert, K.; Elliott, M.; Jenssen, H.; Kindrachuk, J.; Fjell, C. D.; Korner, J.; Winkler, D. F. H.; Weaver, L. L.; Henklein, P.; Ulrich, A. S.; Chiang, S. H. Y.; Farmer, S. W; Pante, N.; Volkmer, R.; Hancock, R. E. W. Chem. Biol. 2009, 16, 58 *and* Reddy, K. V.; Yedery, R. D.; Aranha, C. Int. J. Antimicrob. Agents 2004, 24, 536*., and a)* Perron, G. G.; Zasloff, M.; Bell, G. Proc. Biol. Sci. 2006, 273, 251*. b)* Van't Hof, W.; Veerman, E. C.; Helmerhost, E. J.; Amerongen, A. V. Biol. Chem. 2001, 382, 597 *and a)* Bagheri, M.; Beyermann, M.; Dathe, M. A. Antimicrob. Agents Chemotherap. 2009, 53, 1132*. b)* Haynie, S.L.; Crum, G. A.; Doele, B. A. Antimicrob. Agents Chemotherap. 1995, 39, 301*. c)* Ferreira, L.; Zumbuehl, A J. Mater. Chem. 2009, 19, 7796 *and a)* Humblot, V.; Yala, J-F.; Thebault, P.; Boukerma, K.; Hequet, A.; Berjeaud, J-M.; Pradier, C-M. Biomater. 2009, 30, 3503*. b)* Gao, G.; Yu, K.; Kindrachuk, J.; Brooks, D. E.; Hancock, R. E. W.; Kizhakkedathu, J. N. Biomacromol. 2011, 12, 3715*. c)* Gabriel, M.; Nazmi, K.; Veerman, E. C.; Amerongen, A. V. N.; Zentner, A. Bioconj.Chem. 2006, 17, 548*. d)* Hilpert, K.; Elliott, M.; Jenssen, H.; Kindrachuk, J.; Fjell, C. D.; Korner, J.; Winkler, D. F. H.; Weaver, L. L.; Henklein, P.; Ulrich, A. S.; Chiang, S. H. Y.; Farmer, S. W; Pante, N.; Volkmer, R.; Hancock, R. E. W. Chem. Biol. 2009, 16, 58*. e)* Steven, H.D.; Hotchkiss, J. H. J. Appl. Polym. Sci. 2008, 110, 2665*. f)* Appendini, P.; Hotchkiss, J. H. J. Appl. Polym. Sci. 2001, 81, 609*. g)* Cho, W. M.; Joshi, B. P.; Cho, H.; Lee, K. H. Bioorg. Med. Chem. Lett. 2007, 17, 5772*).* To the best of our knowledge, not many studies have been done to demonstrate the antimicrobial activity of immobilized AMPs in the presence of human serum, except one study where the AMP conjugated with polymer brush on the titanium surface was showed to have excellent antimicrobial activity in vivo (Gao, G.; Lange, D.; Hi/pert, K.; Kindrachuk, J.; Zou, Y.; Cheng, J. T. J.; Kazemzadeh-Narbat, M.; Yu, K.; Wang, R; Straus, S. K.; Brooks, D. E.; Chew, B. H.; Hancock, R. E. W.; Kizhakkedathu, J. N. Biomater. 2011, 32, 3899). Disclosed herein are techniques that have been developed to provide robust coatings of antimicrobial peptide (AMP) on various surfaces with antimicrobial activity in the presence of human serum. Cecropin-mellitin (CM) with cysteine at C-terminal was chosen as the antimicrobial peptide to be immobilised on the gold nanoparticle coated glass and titanium surfaces. CM peptide is a hybrid antimicrobial peptide with 15 amino acid lengths, containing sequences from cecropin-A and mellitin antimicrobial peptides. Cystamine and thiol-PEG-amine were used to functionalize surfaces to study the effect of chain length and flexibility of both molecules on antimicrobial activity of the tethered CM peptide. A large amount of CM peptide per centimeter square (1.02 mg/cm2) was immobilized on the thiol-PEG-amine functionalized surface compared to other reported method ((b) Gao, G.; Yu, K.; Kindrachuk, J.; Brooks, D. E.; Hancock, R. E. W.; Kizhakkedathu, J. N. Biomacromol. 2011, 12, 3715. c) Gabriel, M.; Nazmi, K.; Veerman, E. C.; Amerongen, A. V. N.; Zentner, A. Bioconj.Chem. 2006, 17, 548 and Gao, G.; Lange, D.; Hi/pert, K; Ifindrachuk, J.; Zou, Y.; Cheng, J. T. J.; Kazemzadeh-Narbat, M.; Yu, K.; Wang, H.; Straus, S. K.; Brooks, D. E.; Chew, B. H.; Hancock, R. E. W.; Kizhakkedathu, J. N. Biomater. 2011, 32, 3899 and Kazemzadeh-Narbat, M.; Kindrachuk, J.; Duan, K.; Jenssen, H. ; Hancock, R. E. W.; Wang, R. Biomater. 2010, 31, 9519). A small amount of CM peptide tethered on cystamine-functionalized surface acts as bacteriostatic while a large amount tethered on cystamine or thiol-PEG-amine functionalized surface acts as bactericidal, which is supported by AFM analysis. The CM peptide tethered on the gold nanoparticle coated titanium surfaces maintains antimicrobial activity in the presence of human serum with an excellent reusability for five cycles.

US2008/292671 discloses an antimicrobial coating comprising poly-L-lysine and silver nanoparticles that is suitable to be bound to the surface of articles. However, the document does not disclose metal nanoparticles bound to said antimicrobial peptide wherein said nanoparticles are metal nanoparticles or a nanoparticle covered by a metal layer, and wherein said metal is gold, titanium, or mixtures thereof.

WO2007/095393 discloses a medical device coated with an antimicrobial peptide, including cecropin-mellitin with cysteine at the C-terminal. Also disclosed is the use of PEG tethers, as are the use of thiol or amine groups for reacting and binding with the substrate. However, metal nanoparticles or nanoparticles with a metal coating, that are functionalized by cysteine and thiol-PEG-amine, wherein the metal is gold, titanium, or mixtures thereof are not taught.

WO2010/014940 discloses conjugates of the peptide antibiotic polymyxin B with gold nanoparticles, which are tested for their antimicrobial activity.

### Summary

The present invention relates to an antimicrobial coating composition for coating articles comprising:
a cecropin-mellitin peptide wherein said cecropin-mellitin peptide has a cysteine at C-terminal;
a nanoparticle bound to said cecropin-mellitin peptide, wherein said nanoparticle is a metal nanoparticle or a nanoparticle covered by a metal layer, and wherein said metal is gold, titanium, or mixtures thereof;
a thiol-PEG-amine;
wherein the nanoparticle is functionalized by cysteine and thiol-PEG-amine and wherein the composition is suitable to be bonded to the surface of the article.

The invention also relates to an article coated with the above antimicrobial coating composition.

The nanoparticles are metal nanoparticles or a nanoparticle covered by a metal layer, wherein the metal is gold or titanium, or mixtures thereof, and the nanoparticles have a size between 10-100 nm, preferably 15-50 nm, more preferably 20-30 nm.

In another embodiment of the antimicrobial coating composition described, the composition may further comprises a binder, more preferably a polymer, even more preferably may be a polycation or a polyanion. In a preferably embodiment said polymer may be a polydopamine.

In another embodiment of the antimicrobial coating composition described, the binder may be bonded to the surface of the article by heating.

In another embodiment of the antimicrobial coating composition described, the composition may further comprises a buffer, preferably selected from the following group: TAPS, Bicine, Tris, Tricine, TAPSO, HEPES, TES, MOPS, PIPES, Cacodylate, SSC, MES.

In another embodiment of the antimicrobial coating composition described, the antimicrobial peptide concentration may be between 0.5 and 1 mM, preferentially between 0.20 and 0.10 mM.

In another embodiment of the antimicrobial coating composition described could be use in medicine, preferentially to coated an article with the antimicrobial composition described. More preferably said article is a medical device, even more preferably said medical device is an implant, a tongue depressor, a medical thermometer, a blood sugar meter, a medical robot, a microchip implant, a surgical tool or a neuroprosthesis.

Another aspect relates to a process for coating articles with the antimicrobial coating composition descried comprising the following steps:
- heating an aqueous solution comprising chloroauric acid;
- addition of sodium citrate, and heating the mixture;
- cleaning the article;
- dipping the article in a solution that comprises an antimicrobial peptide for functionalized the article;
- immersing the functionalized article in said heated mixture.

### Description of the drawings

The figures which fall under the scope of the claims represent embodiments of the disclosure subject matter, all others are reference figures
Figure 1: Time dependent UV-vis spectra of gold nanoparticles synthesized in the presence of CMC peptide at HH (A), HW (B) and WH (C) and WW (D) conditions.
Figure 2: Time dependent UV-vis spectra of gold nanoparticles synthesized using 200 HEPES in the presence of CMC peptide (A) and 100 mM HEPES in presence of CM peptide (B). (C) UV vis spectra of gold nanoparticles synthesized using 100 HEPES in absence of peptide (curve 1), in the presence of 0.25 mM cysteine (curve 2) and 0.25 mM cysteine and CM peptide (curve 3). (D) UV-vis spectra of gold nanoparticles synthesized in the presence of CMC peptide using 100 mM HEPES at pH 5 (curve 1), pH 6 (curve 2) and pH 7.5 (curve 3).
Figure 3: Representative TEM images of gold nanoparticles synthesized using 100 mM HEPES in HH (A), HW (B) WH (C) conditions and (D) 200 mM HEPES in HH condition in the presence of CMC peptide. (E) TEM image of gold nanoparticles synthesized using 100 mM HEPES in the absence of peptide. (F) TEM image of gold nanoparticles synthesized using 100 mM HEPES buffer in the presence of CM peptide.
Figure 4: Representative TEM images of gold nanoparticles synthesized using 100 mM HEPES buffer in presence of cysteine (A) and cysteine and CM peptide (B). Gold nanoparticles synthesized using 100 mM HEPES at pH 5 (C) and pH 6 (D) in the presence of CMC peptide.
Figure 5: (A) TGA analysis of gold nanoparticles synthesized in absence of CMC peptide (curve 1), and the presence of CMC peptide in WH (curve 2), HW (curve 3) and HH (curve 4) conditions. (B) FTIR analysis of CMC peptide (curve 1) and gold nanoparticles synthesized in HH (curve 2), HW (curve 3), WH (curve 4). Curve 5, Figure 5B corresponds to FTIR spectrum of gold nanoparticles synthesized using HEPES buffer in the absence of CMC peptide. Antimicrobial activity of different amount of CMC peptide synthesized Au NPs against E.coli (C) and S.aureus (D).
Figure 6: (A) UV-vis spectra of HH-Au NPs (curve 1), after incubated with 10% human serum (curve 2) and 1 mg/mL trypsin (curve 3). An inset of Figure A shows UV-vis spectra of thiol-PEG functionalized citric acid reduced Au NPs (curve 1) and after incubated with 10 % human serum (curve 2). (B) Antimicrobial activity test of HH-Au NPs treated with human serum and trypsin and CMC peptide treated with trypsin against E.coli.
Figure 7: Particle size analysis of gold nanoparticles synthesized in the presence of CMC peptide using 100 mM HEPES (pH 7.5) in HH (A), HW (B) and WH (C) conditions, and 200 mM HEPES (pH 7.5) (D). Particle size analysis of gold nanoparticles synthesized in the presence of CM peptide (E), cysteine (F) and cysteine and CM peptide (G). Particle size analysis of gold nanoparticles synthesized in the presence of CMC peptide using 100 mM HEPES at pH 5 (H) and pH 6 (I).
Figure 8: Representative low (A) and high magnification (B) TEM images of gold nanoparticles synthesized in the presence of CMC peptide at pH 5.
Figure 9: Antimicrobial activity test of different concentrations of CMC peptide against E.coli.
Figure 10: Study of antimicrobial activity of HH-Au NPs stored for different periods and reusability against E.coli.
Figure 11 - (A) The UV-Vis spectrum of gold nanoparticle solution (B) TEM image of gold nanoparticles synthesized using citrate method (C) AFM image of the gold nanoparticle coated glass surface.
Figure 12 - (A) Quantification of amine groups on cystamine and thiol-PEG-amine coated surfaces at different incubation time (B) Estimation of immobilized CM peptide on gold nanoparticle coated surface (curve 1), cystamine (curve 2), thiol-PEG-amine (curve 3), cystamine-sulfo GMBS (curve 4) and thiol-PEG-amine-sulfo GMBS (curve 5) functionalized gold coated surfaces.

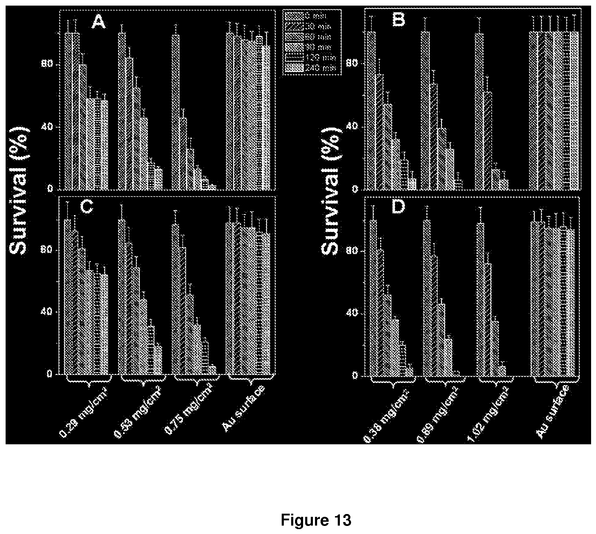

Figure 13 - Antimicrobial activity of CM peptide coated on CS and NS surfaces against Escherichia coli (A and B) and Staphilococcus aureus (C and D).

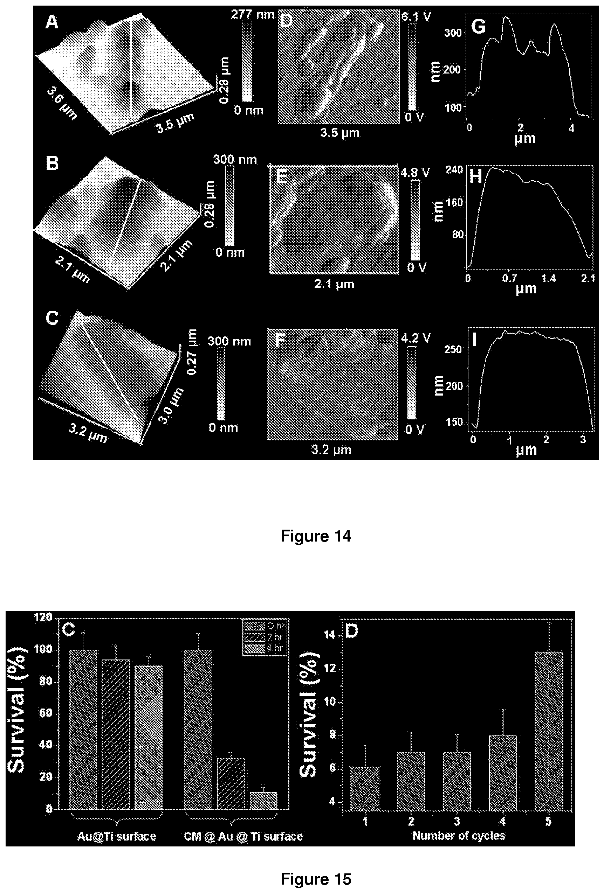

Figure 14 - Representative height mode AFM images of Escherichia coli treated with CM peptide immobilized NS (A), CS (B) surfaces and control (E. coli has grown in solution).
Phase mode images (D-F) and cross sectional analyses (G-I) of corresponding height mode images (A-C).
Figure 15 - (A) Antimicrobial activity of randomly attached CM peptide on Au NPs coated surface. (B) Antimicrobial activity of CM peptide attached on different ratios of thiol-PEG-amine and thiol-PEG-acid modified Au NPs surfaces. (C) Antimicrobial activity of CM peptide attached on N:C 1:1 modified Au NPs coated Ti surface. (D) Reusability study of CM peptide attached on N:C 1:1 modified Au NPs coated Ti surface.

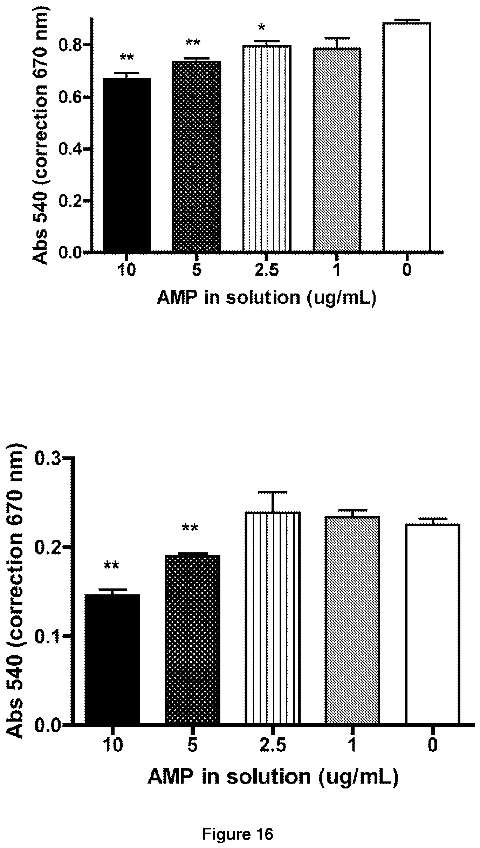

Figure 16 - Effect of soluble CM peptide on human dermal fibroblasts (NDHF) (7A) and human umbilical vein endothelial (HUVEC) (7B) cells cytotoxicity. * p<0.05.

### Detailed description of the invention

The invention relates to an antimicrobial coating composition for coating articles comprising an antimicrobial peptide; a nanoparticle bound to said antimicrobial peptide; wherein the composition is suitable to be bonded to the surface of the article.

More preferably: the nanoparticles are suitable to be bonded to the surface of the article by heating, or the composition further comprises a binder for attaching the composition to the surface of the article.

In the present subjecmater, the impact of antimicrobial peptide (ex: cecropin melettin) and buffer (ex:HEPES - (4-(2-hydroxyethyl)-1-piperazineethanesulfonic acid)) concentrations, pH of buffer and peptide chain on the gold nanoparticle formation process was studied under ambient conditions using experimental and molecular modeling approaches. Cecropin melettin (CM) peptides with and without cysteine at C-terminal are used for the synthesis of gold nanoparticles. It is found that thiol group of cysteine residue plays a critical role in controlling the size and morphology of gold nanoparticles via the digestive ripening and surface reactivity processes. The CM peptide capped gold nanoparticles have excellent properties of killing both Gram-positive and Gram-negative bacteria in MIC range of 10 µg/mL in the presence of human serum along with resistance to protease degradation and biocompatibility to human umbilical vein endothelial (HUVEC) and normal human dermal fibroblast (NDHF) cells up to concentration of 100 µg/mL.

Furthermore, bacterial colonization and subsequent infections are believed to be one of the main reasons for the failure of biomedical implants. There are several coatings approaches using antibiotics, quaternary ammonium salts, polycation and photosensitiser antimicrobial materials, however none of them succeeded due to less antimicrobial activity and more cytotoxicity effect. Herein is described a procedure for providing a robust coating on gold nanoparticle coated glass and titanium surfaces using cecropin-melittin (CM) peptide. The CM peptide was covalently grafted on cystamine and thiol-PEG-amine functionalized surfaces using bilinker sulfo-GMBS with maintenance of its potent antimicrobial activity. A small amount of tether CM peptide on cystamine-sulfo GMBS functionalized surface acts as bacteriostatic while a large amount of tethered CM peptide on both surfaces acts as bactericidal. The CM peptide tethered on binary self-assembled monolayers of thiol-PEG-amine and thiol-PEG-acid has potent antimicrobial activity in the presence of 20 % human serum with excellent reusability up to 5 cycles.

### NANOPARTICLE PREPARATION AND CHARACTERIZATION

### MATERIALS

HAuCl4.3H2O, NaH2PO4, Na2HPO4 (preferably, Sigma-Aldrich) and HEPES (preferably, Anal Chem) were used as received. Cecropin melettin with cysteine (KWKLFKKIGAVLKVLC) and without (KWKLFKKIGAVLKVL) peptides, preferably from Caslo Laboratory, Denmark. Human Serum AB, preferably, from Invitrogen.

### METHODS

0.5 mM CMC (Cecropin melettin peptide with cysteine) and CM (Cecropin melettin without cysteine) Peptides were dissolved initially in 50 µL DMF followed by addition of 950 µL HEPES (100 mM, pH 7.5) or milli-Q water. Gold ions were added in peptide solutions (0.25 mM) to get the final gold concentration of 0.5 mM at different conditions for the synthesis of gold nanoparticles at 25 °C (Table SI 1):

**Table SI 1: Different conditions used for the synthesis of gold nanoparticles in the presence of CMC peptide.**

| **Conditions** | **AMP (volume)** | **AMP (Final concentration)** | **AuCl₄⁻** | **Solvent** | **Final volume** |
|---|---|---|---|---|---|
| HH | AMP in HEPES (468 µL) | 0.25 mM | 50 µL | HEPES (482 µL) | 1000 µL |
| HW | AMP in HEPES (468 µL) | 0.25 mM | 50 µL | HEPES (482 µL) | 1000 µL |
| WH | AMP in Water (468 µL) | 0.25 mM | 50 µL | Water (482 µL) | 1000 µL |
| WW | AMP in Water (468 µL) | 0.25 mM | 50 µL | Water (482 µL) | 1000 µL |

Time dependent UV-vis spectra were recorded, preferably, using BioTek synergy MX microplate reader to monitor the reduction of gold ions. Gold nanoparticles were also synthesized using HEPES with similar concentration of gold ions without peptides. To understand the effect of thiol group, 0.25 mM cysteine dissolved in HEPES was added in reduction solutions containing gold ions (0.5mM) and HEPES (100mM) or gold ions, HEPES and CM peptide (0.25 mM) at HH condition. Transmission electron microscopy (TEM) measurements of gold nanoparticle samples were carried out on Jeol A minimum of 100 nanoparticles was measured using Image J software for the particle size analysis. The synthesized gold nanoparticle solutions were centrifuged at 14000 rpm followed by washing with mill-Q water to remove unreacted peptides and HEPES. The centrifuged gold nanoparticles were frozen and fridge dried at 223 K. Thermogravimetric analysis (TGA) of peptide capped gold nanoparticles and gold nanoparticles synthesized without peptides was performed using over a temperature range of 30-600 °C at a heating rate of 10 °C /min in presence of N2 gas. The binding of peptide on gold nanoparticle surface was analyzed, preferably by FTIR spectroscopy in ATR mode using JASCO spectrophotometer at 4 cm-1 resolutions with 64 scans. Zeta (ζ) potential of the synthesized gold nanoparticles after centrifugation and redispersion in HEPES were measured, preferably, using Brukerheaven instrument at room temperature.

Antimicrobial Activity Testing:
S. aureus and E. coli were grown at 37 °C and maintained on LB plates (preferably, Luria-Bertani broth with 1.5% agar). Bacteria were also grown in LB media for overnight at 37 °C and cell counts were quantified by OD 600 measurements. Different amounts of peptide capped gold nanoparticles, peptide (5-10 µg/mL) and HEPES reduced gold nanoparticles (10 µg/mL) were added into bacterial suspensions (105 bacteria/mL) in presence of 10 % human serum and incubated for 4 h at 37 °C. 100 µL aliquots were taken out from the respective suspensions at 2 h intervals and diluted in PBS buffer to give 103 bacteria per mL and plated on LB agar plates followed by incubation at 37 °C. Colonies were counted after 24 h of incubation. In another experiments, citric acid reduced gold nanoparticles were functionalized with 1 mg/mL mPEG-thiol (5 kDa). Gold nanoparticles were synthesized using citric acid as described previously (11). PEG and CMC peptide coated gold nanoparticles were incubated with 10 % human serum for 2 hr at 4 °C followed by centrifugation at 14000 rpm to discard free human serum. Similarly, to study protease degradation, CMC coated gold nanoparticles and bare CMC peptides were treated with 1 mg/mL trypsin (phosphate buffer pH 8) for 2 h at 37 °C. After 2 h of reaction, the nanoparticle solution was subjected to centrifugation to remove trypsin. Antimicrobial test was performed with human serum and trypsin treated peptide capped gold nanoparticles against E.coli.

### Results:

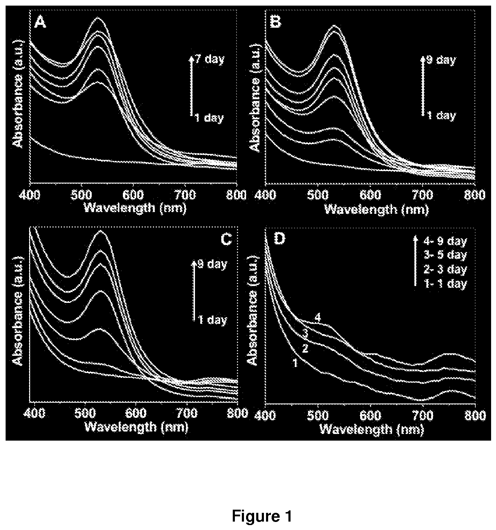

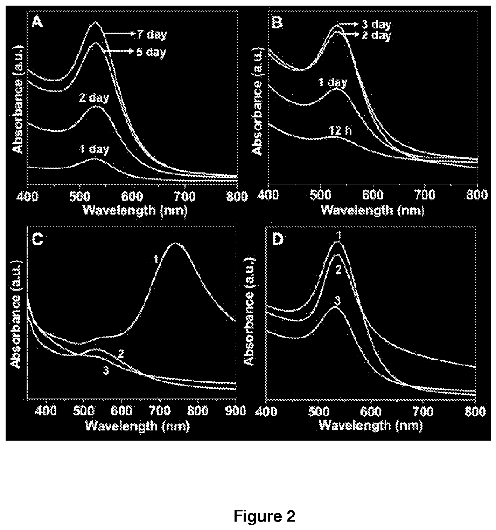

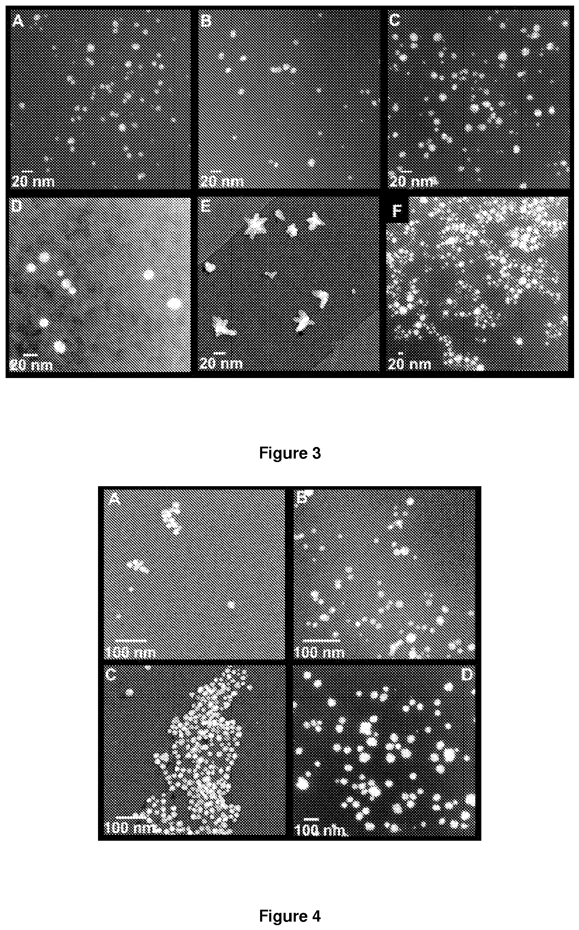

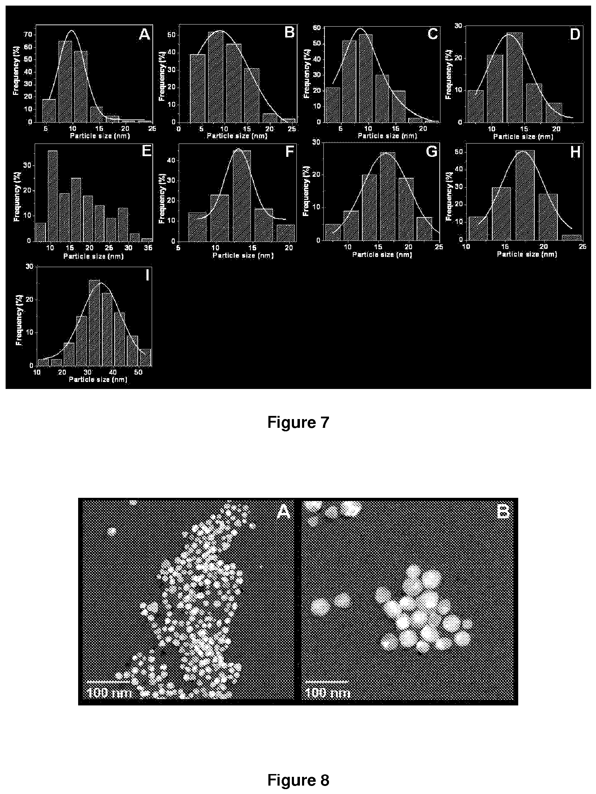

The growth kinetic of gold nanoparticles synthesized under different conditions (HH, HW, WH and WW) using HEPES buffer (100 mM and pH 7.5) and cecropin melletin (CMC) peptide (0.25 mM) at 25 °C was followed by UV-Vis spectroscopy. Figure 1 shows UV-Vis spectra of gold nanoparticle solutions synthesized as a function of reaction time. UV-Vis spectra clearly show an intense surface plasmon resonance (SPR) band at 530 nm after 7 days of reaction for all conditions (Figure 1A-C). The SPR band at 530 nm signifies the synthesis of spherical gold nanoparticles with size ranging from 9 to 14 nm in all conditions (Figure 3A-C). The rate of reduction of gold ions was faster in HH condition with saturation being reached in 7 days compared to HW and WH conditions where the saturation reached in 9 days (Figure 1A-C). The gold ions were reduced by excessive amount of HEPES, resulting into appearance of solution from light yellow to pink after 2 days in all conditions. The thiol groups of CM peptides form complex with Au(0) atoms through electrostatic and/or coordinate interactions at initial stage of reaction, leading to delay in formation of nuclei and subsequently nanoparticle growth^{9a, 9c}. This phenomenon could be explained by inhibition of the surface reaction, which act as growth limiting step, in which CMC peptide-Au(O) complexes retard the surface reaction with gold ions to form nuclei^{9c, 12}. It is worth to mention here that the diffusion-limited mechanism does not relevant in our case as supported by others for the formation of monodisperse gold nanoparticles using HEPES and peptides^{9a,b,12}. If this mechanism exists, then a higher concentration of HEPES should synthesize gold nanoparticles at faster rate owing to the presence of large amount of N-centered cationic radicals compared to 100 mM HEPES case. Interestingly, no change in rate of reduction of gold ions (7 days) and the SPR band position (530 nm) were observed with 200 mM HEPES, which explain that the surface reactivity of nuclei controls the growth rate (Figure 2A). The critical concentration of free Au (0), peptide and peptide-Au(O) complex in reaction solution is key determinants in controlling the growth and size of nanoparticles. For instance, no synthesis and aggregation were found in reaction solutions when the peptide concentrations were varied above (such as 0.5 and 1 mM) or below the 0.25 mM (such as 0.20 and 0.10 mM) respectively with the constant concentration of gold ions (0.5 mM) in HH condition. In addition, size focusing and digestive ripening processes catalyzed by thiol group of peptides have significant contribution in reduction of the particle size and polydispersity over a long reaction period (7 days) 13 as mirrored in transformation of broad to narrow SPR band with time (Figure 1A-C). Supporting evidence for this observation was obtained by TEM analysis of nanoparticles synthesized at different conditions (HH, HW and WH with 100 mM HEPES). TEM and particle size analyses revealed that the size of spherical gold nanoparticles was approximately 9-14 nm for all conditions, although a small population of larger nanoparticles (20-25 nm) was also observed (Figure 3A-C and 7A-C). Similarly, the spherical gold nanoparticle of mean size 13 nm was synthesized using 200 mM HEPES (Figure 3D). In contrast, peptides alone (without HEPES in WW condition) have no ability to reduce the gold ions (Figure 1D) even after 9 days of reaction, indicating that the HEPES and the peptide act as reducing and capping agents respectively (9a-c). In the absence of peptide, the gold ions were reduced rapidly by HEPES within 30 min with the SPR band centered at 740 nm, as also observed by other (curve 1, Figure 2C)14. The redox mechanism of HEPES molecule has been studied in details previously in which N centered cationic free radicals are formed after N substituted piperazine ring of HEPES being oxidized by the gold ions.

To understand the role of peptide chain in nanoparticle formation process, the same sequence of peptide without thiol group (CM peptide) was used for the synthesis of gold nanoparticles. The synthesis of gold nanoparticles in HH condition was comparably fast with the reduction being saturated within 2 days, albeit red solution started appearing after 12 hr of reaction (Figure 2B). The UV-Vis spectrum showed a SPR band at 530 nm similar to the gold nanoparticles synthesized using CMC peptide (Figure 1 and 2B). TEM images and particle size analysis showed polydispersed spherical gold nanoparticles of size ranging from 8 to 35 nm (Figure 3F and 7E). The different dispersity in size of the gold nanoparticles for both CMC and CM peptides is consistent with their different interfacial binding strength with Au(0) and the presence and absence of digestive ripening process respectively. Since the CM peptides passivate Au(0) modestly via gold-amine interactions, the retardation of surface reaction is mild and it only slow down the growth rate slightly, but not chelate sufficient number of Au(0) to form peptide-Au(O) complex in order to alter substantially nuclei formation rate. HEPES molecules rapidly convert gold ions to Au(0), so the initial concentration of Au(0) is higher in the reaction solution. The growth of polydisperse gold nanoparticles occurs via a multistep growth mechanism. The small nanoparticles form first in solution in the presence of peptide, which limits the growth to below 12 nm and yield a narrow size distribution of this population. Larger gold nanoparticles may form via the aggregation of smaller nanoparticles as suggested by others (15). Alternatively, the most of peptides were adsorbed on the surface of smaller nanoparticles; larger nanoparticles (30 nm) may grow with a lesser restriction in terms of time and size imposed on them by fewer peptides. Moreover, the polydispersity is suggestive of no occurrence of digestive ripening process due to the absence of thiol group in the CM peptide.

In fact, when cysteine (0.25 mM) was added in solution containing HEPES and gold ions without peptides, it was observed that the synthesis of gold nanoparticles was rapid (30 min) similar to reaction without cysteine (curve 1 and 2, Figure 2C). However, the SPR band position drastically blue-shifted to 530 nm and the spherical gold nanoparticles of mean size 13 nm were synthesized in comparison with multi-branched gold nanoparticles formed without cysteine (Figure 3E and 4A). In another case, when a similar molar concentration of cysteine was added in reaction solution containing 0.25 mM CM peptide, the reducing ability of HEPES molecules was almost retarded; a weak and broad SPR band was observed upon synthesis of spherical gold nanoparticles of mean size 17 nm after 9 days of reaction (curve 3, Figure 2C and Figure 4B). These data suggest that thiol group preferentially bind to surface of the gold nuclei formed in solution followed by binding of the peptide instead of excess amount of HEPES. Thiol groups do not interfere in reduction of the gold ions by HEPES but the combined action of thiol and peptide chain plays an important role in controlling the growth rate, size and shape of nanoparticles, which is in agreement with prior conclusion with CMC peptide (Figure 3 and 4).

The pH of HEPES buffer also affects the reduction rate and the size of nanoparticles. The varying pH of reaction solution alters the charge of CMC peptide and the speciation of gold ions, which influence significantly the affinity of peptide to Au(0), resulting into a disproportionate ratio of critical concentration of peptide-Au(O) complex and free Au(0) in the reaction solution. With decreasing pH of HEPES buffer, the reduction time of gold ions changed drastically to 24 hr and finally to 1 hr for pH 6 and 5 respectively (Figure 2D). On the other hand, aggregation of the gold nanoparticles was observed above pH 7.5 (pH 8 and 9) within 1 hr of reaction (data not shown). UV-vis spectra show that the SPR bands of gold nanoparticles were red shifted to 540 nm for pH 5 and 6 in comparison with pH 7.5 (Figure 2D). A red shift in the SPR band is symptomatic of increase in the size of gold nanoparticles. This result is in agreement with TEM analyses, which show the synthesis of nanoparticles of mean sizes 17 nm and 35 nm for pH 5 and 6 respectively (Figure 4C,D and 7H,I). TEM images clearly show the formation of large population of multi-shaped (triangles, hexagons and random) gold nanoparticles at pH 5 and 6 due to the absence of digestive ripening process in limited reaction time (1 and 24 hr respectively) at 25 °C (Figure 4C,D and 8). Digestive ripening process was used to transform polydisperse and prismatic nanoparticles to narrow size distributed spherical nanoparticles using alkanethiol under boiling condition in 90 min (16). After comparing the obtained data with results from previous papers (9a-c,17) there is no experimental baseline in terms of concentration of HEPES/peptide and the pH of reaction solution to synthesize gold nanoparticles using different peptides.

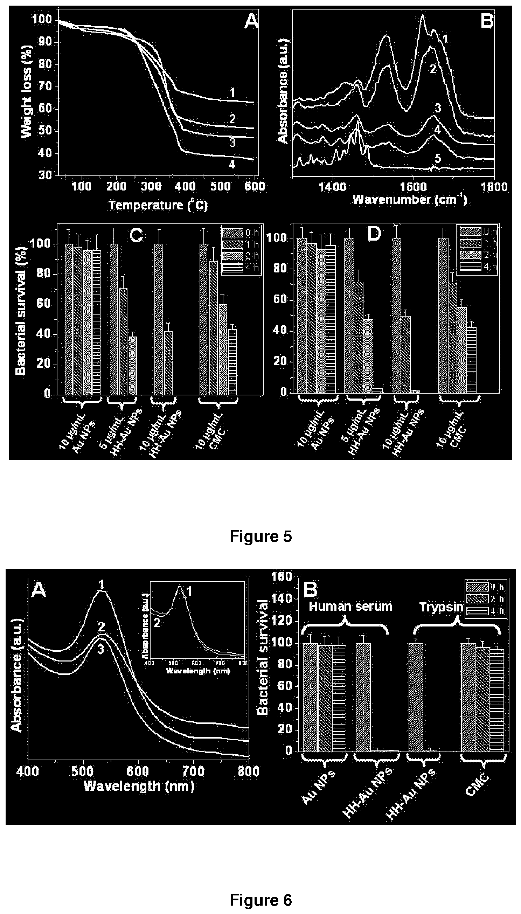

Thermogravimatric analysis (TGA) of the gold nanoparticles synthesized in different conditions was performed to obtain quantitative information of the peptide content. The initial 2 to 8% weight loss in temperature region of 30 to 240 °C is due to desorption of water molecules from samples (Figure 5A). The weight loss of 26% is found for HEPES reduced gold nanoparticles in multiple distinct steps until 480 °C with initial gradual and then rapid loss at higher temperature. In case of peptide capped gold nanoparticle samples, there is a rapid weight loss until 400 °C with mass decreased of 55, 43 and 38% for the gold nanoparticles synthesized in HH, HW and WH conditions respectively (Figure 5A). The variation in weight loss is due to the fact that a large number of gold nanoparticles synthesized in the HH condition consume a large amount of peptide compared to other conditions, leading to varying densities of peptide on the nanoparticle surface (Table 1). The number of nanoparticles was calculated using the equation proposed by Haiss et. Al (18). The number of peptides bound to each nanoparticles is determined using TGA analysis as described previously (9d,19). The surface charge of gold nanoparticles synthesized in all conditions is positively charged (+ 28 ± 2 mV) similar to native peptide (+ 23 ± 1 mV), confirming the presence of CMC peptides on the surface of nanoparticles and the colloidal stability of nanoparticle solution (Table 1):

**Table 1: Estimation of number and surface charge and peptide density of gold nanoparticles synthesized in different conditions.**

| Condition | Number of nanoparticles | Peptide/nanoparticle | Peptide/nm2 | ζ potential (mV) |
|---|---|---|---|---|
| HH | 5.8 x 1015 | 556 | 1.24 | +28 ± 2 |
| HW | 1.1 x 1015 | 466 | 1.03 | + 27.5 ± 2.2 |
| WH | 0.98 x 1015 | 453 | 1.01 | + 26.1 ± 1.6 |

To compliment TGA analysis, FTIR spectroscopy was performed to analyze the binding of peptide on the gold nanoparticle surface. Curve 1, Figure 5B shows the characteristic amine-I band at 1623 and 1650 cm-1, corresponding to predominantly β-sheet and α-helix secondary structures of the CMC peptides. The CMC peptides have different ratios of β-sheet and α-helical structures, depending on the amino acid compositions^{7a, b, 20}. However, the CMC peptide capped on the surface of gold nanoparticles showed random coil structures (amine-I band at 1646 cm-1) as AMPs orient into random coil structure in aqueous solution (Curves 2-4, Figure 5B)^{7a, 20-21}. No signature of the peptide was found for the HEPES synthesized nanoparticles in the absence of CMC peptide (Curve 5, Figure 5B), which is concomitant with the surface charge of nanoparticles (- 33.5 mV).

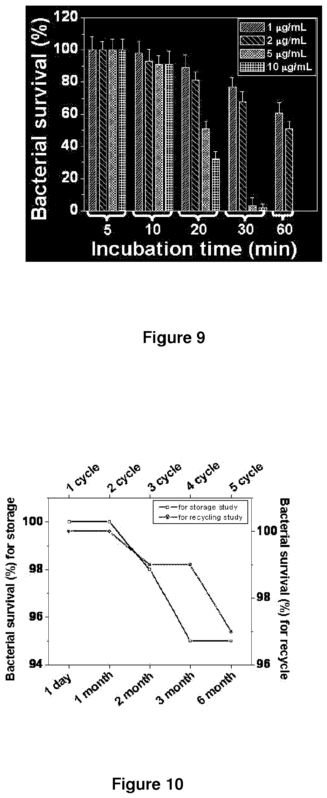

The CMC peptides adopt α-helical structure in the presence of membrane or helix promoting organic solvents, which triggers them to act as a potent antimicrobial agent20, 22. In order to validate the antimicrobial activity of synthesized gold nanoparticles in the HH condition (HH-Au NPs), HH-Au NPs were tested against Gram-positive (S. aureus) and Gram-negative (E. coli) bacteria for different incubation times in the presence of 10% human serum to mimic in-vitro tissue culture condition. The gold nanoparticles synthesized in the HH condition were chosen due to high loading of peptide. The amount of HH-Au NPs used for antimicrobial test was taken in the range of minimum inhibitory concentration (MIC) of CMC peptide (5 µg/mL) as reported previously (Figure 9) . As incubation time increases, colony forming unit (CFU) of both bacteria decreased with increasing amount of HH-Au NPs (Figure 5C and D). Figure 5C and D show that no bacterial colony was observed for the 10 µg/mL HH-Au NP solution after 2 hr of incubation. The killing activity of 5 µg/mL HH-Au NPs was a little slow and the 100 % antimicrobial activity was found after 4 hr of incubation. Thus, the MIC of HH-Au NPs is 10 µg/mL for both bacteria. On the other hand, 10 µg/mL of HEPES reduced gold nanoparticles (Au NPs) did not kill bacteria even after 4 hr of incubation (Figure 5C and D). It is well known that PEG modified surfaces prevent the adsorption of proteins. PEG layer might provide an interfacial layer to prevent the proteins to get adsorbed on the surface of underlying nanoparticles. This unique property of PEG depends on molecular weight, chain length and interfacial chain density of PEG molecules. The HH-Au NPs behave in a similar manner to PEG coated gold nanoparticles to resist the adsorption of human serum on the surface of the gold nanoparticle with maintenance of antimicrobial activity²³. To validate this hypothesis, the HH-Au NPs and PEG coated gold nanoparticles (PEG-Au NPs) were incubated in 10% human serum for 2 hr. A sharp SPR band along with no shift in the band position was observed for both gold nanoparticles compared to HH-Au NPs and PEG-Au NPs without treatment, which indicate that the protein monolayer does not form on the nanoparticle surface (curves 1 and 2, Figure 6A and inset of Figure 6A). It could be possible that protein submonolayer was formed on surface of the gold nanoparticles, which was not detected with UV-vis spectroscopy23b. Human serum treated HH-Au NPs (10 µg/mL) killed 99 % bacteria (E. coli) in 2 hr of incubation in line with our previous experiment (Figure 5C,D and 6B). In comparison to HH-Au NPs, the reduced activity of CMC peptides was observed in the presence of human serum after 4 hr of incubation even using 10 µg/mL, which is twice the MIC of the CMC peptide (Figure 5C and D). The CMC is a very potent peptide to kill bacteria in 60 min of incubation in the absence of human serum, indicating that human serum drastically reduces the activity of the peptide (Figure 9).

The highly cationic CMC peptides first associate with bacterial membrane via electrostatic interactions and then peptides, transformed into α-helical structures after being contacted with membrane, span through the lipid bilayer^{7e, 21-22}. The long chain peptide expands the membrane surface upon insertion to create pores^{21, 22b}. It's suggest that the combined action of peptides to form pores/ion channels and the gold nanoparticles to generate holes raptures completely the bacterial membrane, which leads to leakage of cell content and finally cell death. In synergy with above action, the inserted gold nanoparticles may be bound to cellular contents (DNA) of bacteria and prevent transcription and translation processing to repair the damage^{2a, 4a}.

Trypsin, a lytic protease found in living system, can degrade AMPs thereby limiting their potential to become next-generation antibiotics. There is several reports in which expensive and tedious methods were adopted to synthesize different analogies of CM peptides in order prevent their degradation from trypsin^{7e, 21, 24}. In contrary, the HH-Au NPs are resistance to 1 mg/mL trypsin and showed no sign of aggregation after 2 hr of incubation that can be attributed to nanoparticles associated protection of peptides from proteases (curve 3, Figure 6A)^{4f,8}. No reduction in antimicrobial activity of HH-Au NPs was found against E. coli while the plain CMC peptide was degraded and did not show any antimicrobial activity after trypsin treatment (Figure 6B). The added benefit of CMC peptide capped to gold nanoparticles is their excellent stability and maintenance of bioactivity for 6 months under refrigerated condition (Figure 10). It should be noted that the CMC peptides alone are required to store at -20 °C to preserve their bioactivity. Besides storage property, the HH-Au NPs showed a mild reduction in bioactivity after 5 cycles of repeated use (Figure 10).

### SYNTHESIS OF GOLD NANOPARTICLES AND COATINGS ON GLASS SLIDES

### MATERIALS

All chemicals (cystamine dihydrochloride, sodium citrate, mono sodium phosphate, disodium phosphate, gold (III) chloride trihydrate, BCA kit), preferably from Sigma Aldrich. CM peptide with 98% purity, preferably fromCaslo laboratory (Lyngby, Denmark). Thiol-PEG-amine (1 kDa) and thiol-PEG-acid (1 kDa), preferablyfrom Creative PEG works (Winston Salem, North Caroline, USA). Human serum, preferablyfrom Invitrogen. All media components for bacterial media, preferably from Frilabo, Portugal. All glassware were cleaned with soap solution followed by aqua regia (1 mL HNO3 (15.9 M): 3 mL HCL (12 M)) and rinsed with Milli-Q water. Glass coverslips, preferably from VWR and Menzel and titanium disks. Escherichia coli (E. coli) (ATCC 25922) and Staphylococcus aureus (S. aureus) (ATCC 6538) were used for antimicrobial study.

### METHODS

The gold nanoparticles (Au NPs) were synthesized using Turkevich's method (Turkevich, J.; Stevenson, P. L.; Hillier, J.; Discuss Faraday Soc. 1951, 11, 55. In general, 90 mL of 10-4 M HAuCl₄ solution in Erlenmyer flask was boiled followed by addition of 10 mL of 0.2 gm of sodium citrate. The solution was boiled until the color of solution changed from light yellow to ruby-red. Circular glass slides were rigorously cleaned before use by submerging into piranha solution (30% H₂O₂ + 70% H₂SO₄) for 1 h. Subsequently they were washed with copious amounts of Milli-Q water. The cleaned glass slides were dipped in 1% of 3-amino propyltrimethoxysilane (APTMS) in ethanol for 30 min followed by cleaning with ethanol. APTMS functionalized glass slides were immersed in the gold nanoparticle solution for 24 hr to deposit gold films. The deposited gold nanoparticle coated surfaces were washed twice with Milli-Q water and gently dried with N2 gas. To coat the gold nanoparticles on titanium (Ti) surface, Ti disks were cleaned with H2SO4 solution for 30 min. Other steps were followed as mentioned above.

### Immobilization of CM peptide

Gold nanoparticles coated surfaces were functionalized with 20 mM cystamine and 1 mg/mL thiol-PEG amine (1 kDa) for 24 h followed by washing with water to remove unbound amine molecules. The quantification of amine functional group present on Au NPs coated glass surfaces was quantified using a picrate test as described before (Lee, C. C. Y.; Loudon, G. M. Anal. Biochem. 1978, 94, 60). The extinction coefficient of amine was considered to be 134700 for the estimation of concentration of amine molecules *(*Lee, C. C. Y.; Loudon, G. M. Anal. Biochem. 1978, 94, 60*).* Freshly prepared 1 mg/mL sulfo-GMBS in phosphate buffer (pH 7.2) was incubated with amine-functionalized surfaces for 2 h to modify the amine groups. Picrate test was again performed to quantify the free amine group on the surface. It should be noted that sulfo-GMBS modified cystamine and thiol-PEG-amine surfaces will be called CS and NS surfaces respectively throughout the paper for simplicity to explain the results. Different concentrations of CM peptide (1-3 mg/mL) dissolved in phosphate buffer (pH 7.2) were incubated with CS and NS surfaces for 24 h at 4 °C for covalent immobilization. The unbound peptide was removed by washing twice gently with phosphate buffer. The amount of peptide in remaining peptide solution after the immobilization and washings were quantified using BCA method as per manufacturer instructions (preferably, Sigma Aldrich). The CM peptide covalently immobilized per unit surface area (mg/cm2) on different surfaces was calculated.

### Fourier Transformed Infrared (FITR) study

The different stage of coating processes was characterized using a golden Gate attenuated total reflection (ATR) accessory in a Jasco spectrophotometer. All samples were dried completely before FTIR measurements. Spectra were recorded at resolution of 4 cm-1 with 128 scans being average and then smoothed by 11 points adjacent averaging.

### Contact Angle Measurements

Contact angle measurements were performed by acquiring images of 5 µL water drops on surfaces using. A picture of water drop was taken after immediately to avoid problems related to drying of the drop. Three images of each sample were taken from different areas of the surface, and tangent measurements at the drop surface interface were made and the contact angle calculated. The relationship between functional groups and water contact angle was assessed using the Wenzel equation where the apparent contact angle of a liquid on a surface that depends on the roughness and chemical composition of the surface (Wenzel, R. N. Ind. Eng. Chem. 1936, 28, 988).

### X-ray Photoemission Spectroscopy (XPS) Measurements

### Antimicrobial Activity

Gram positive (S. aureus) and Gram negative (E. coli) were grown in Luria Broth (LB) media for 24 h at 37 °C. 100 µL of bacterial suspensions were transferred in sterile vials containing 5 mL of LB media and incubated for 2 h at 37 °C to get mid logarithmic phase growth of bacteria then further diluted using LB media to achieve 105 CFU/mL bacteria. CM tethered surfaces were incubated in phosphate buffer saline (pH 7.2, PBS) for 4 h to leach out any unbound and physically adsorbed CM peptide. To test the antimicrobial activity of tethered CM peptide, different surfaces with CM peptide were incubated in 105 CFU/mL S. aureus and E. coli suspensions at 37 °C for 4 h. Aliquots (100 µL) were taken out from respective bacterial solutions at 30 min intervals and diluted to 105 CFU/mL in PBS and then plated on LB agar plates followed by incubation at 37 °C. Colonies grown on plates were counted. All antibacterial activity tests were performed in triplicate and were performed at different times to certify the reproducibility of the data. The leached solution was also tested against E. coli and no killing of bacteria was observed. To test the reusability of CM peptide coated glass surface, this surface was incubated in E. coli for 4 h then washed with PBS and air-dried.

To test the antimicrobial activity of tethered CM peptide in the presence of human serum (HS), the above functionalization procedure with thiol-PEG-amine was slightly modified. After functionalization of the Au NPs coated glass surfaces with 1 mg/mL thiol-PEG-amine, these surfaces were further incubated with different amount of thiol-PEG-acid solution for 12 h to get different molar ratios of amine and acid (different molar ratios of amine to acid was denoted as N:C 1:1, 2:1, 3:1, 4.1 and 5:1 in the mansucript). After 12 h, the functionalized surfaces were washed with ethanol to remove unbound molecules. Other steps to immobilize CM peptide on these surfaces were
similar as mentioned above using 3 mg/mL CM peptide. CM tethered N:C surfaces were incubated with 20 % human serum for 2 h followed by washing with sterile PBS. Human serum (HS) was diluted in PBS to get 20% concentration. HS treated N:C surfaces were tested against E. coli. Similarly, Au nanoparticle coated titanium surfaces were immobilized with 3 mg/mL CM peptide using N:C 1:1 surface chemistry. CM immobilized Au nanoparticle coated Ti surfaces were treated with 20 % human serum for 2 hr followed by antimicrobial activity test against E. coli.

### Cell Cultures

Two human cell types such as human endothelial umbilical vein cells (HUVEC) as a model for vascularized tissues and normal dermal human fibroblasts (NDHF) one of the most common cell type present in human connective tissue were used to evaluate the cytotoxicity induced by CM peptide immobilized surfaces. HUVECs (preferably, Lonza, Cat. Number CC-2517, Walkersville, Maryland, USA) were cultured using endothelial growth factor-2 media (EGM-2), bullet kit media (preferably, Lonza, Cat. Number CC-3156) for at least 24 h prior to further use. Passages below six were used for all experiments.

NDHF cells (preferably, Lonza, Cat. Number CC-2511) were cultured with dulbecco's modified eagle's medium (DMEM) (preferably, Sigma, Cat. Number D5796) supplemented with 10% (v/v) of foetal bovine serum (preferably, Gibco, EU approved origin, Cat. Number 10270-106, Grand Island, NY, USA) and 1% (v/v) penicillin/streptomycin (preferably, Lonza, Cat. Number DE17-602E). NDHF cells under passage number ten were used in order to maintain primary cell state during the time of assays performed.

Both HUVEC and NDHF cells were cultured in their respective media in tissue culture flasks with 75 cm2 of surface (BD, Falcon). Cells were trypsinized, centrifuged and counted for adequate assessment of cell number before seeding in the appropriate tissue culture plates for cytotoxicity investigations.

### Soluble CM Peptide Interaction with Cells

HUVEC and NDHF cells were cultured as previously mentioned and platted at a density of 4.2x103 cells/cm2 in 24-well plates. Cells were allowed to grow 24 h prior to any experiments performed. Different concentrations of CM peptide solutions (50, 30, 20, 10, 5, 2.5 and 1 µg/mL) were incubated with both cells for 24 hr to evaluate the cytotoxicity effect of CM peptides using MTT cell metabolic activity assay.

### MTT Assay

Initially cells were washed with warm phosphate buffer saline (PBS). Cells treated with different concentrations of CM peptides after 24 h were incubated with working solution of thiazolyl blue tetrazolium bromide salt (MTT) solution (10 mL of media to 1 mL of stock solution of tetrazolium salt, 5 mg/mL in PBS) for 3 h at 37 °C in the CO2 incubator with 5% air and 95% CO2. After 3h of incubation, the formed purple formazan crystals were dissolved by dimethyl sulfoxide (DMSO) using a gentle agitation of the test plate in an orbital shaker. Absorbance was measured at 540 nm with reference to 670 nm in a UV-visible spectrophotometer (SynergyMx from Biotek Instruments, Winooski, VT, United States) (I don't understand by reference).

In case of the direct contact assay, later on detailed explained, samples were transferred to a new plate before addition of MTT working solution to avoid miscalculation in the cellular activity. Using this strategy it is only taken into account the cells that were in contact with the surface under testing. For TCPS condition, obtained values were normalized for the same surface area as the modified samples to have for all the conditions the same surface area.

### Extraction Assay

Extraction assay was performed to evaluate whether any leached CM peptide from surfaces in media has effect on metabolic activity of both cells. Au NP coated, N:C (1:1) coated and CM peptide immobilized on N:C (1.) coated Au NP surfaces were sterilized for 20 min using UV light source in laminar flow hood before the extracts were prepared. Extracts were prepared according to the international norm ISO 10993-5 and both culture medias were in contact with the previously mentioned surfaces of 3 cm2 area for 24 h in sterile conditions in an incubator at 37 °C with gentle agitation. Media recovered from surface treatment acts as extracts. Cells were seeded at a density of 4.2x103 cells/cm2 and allowed to grow 24 h before the extracts were added to the wells. The ratio of surface area of samples to media was kept constant to 3 cm2/mL in each experiment. Extracts were maintained in contact with the seeded cells for 24 h followed by the assessment of cytotoxicity using MTT assay.

### Direct Contact Assay

Different surfaces sterile under UV light as mentioned above were used for direct contact assay. Both cells were seeded at a density of 4x104 cells/cm2 on the top different surfaces and allowed to grow for 24 h before cytotoxicity was assessed using MTT assay.

Samples were transferred to a new plate in order to avoid miscalculation in the cellular activity measured and to take into account only the cells that were in contact with the surface under testing. For TCPS condition, obtained values were normalized for the same surface area as the modified samples to have for all the conditions the same surface area (I did not understand).

### Scanning Electron Microscopy (SEM)

Different types of modified glass surfaces were used for these analyses: glass coverslips coated with gold nanoparticles as a control and glass coverslips coated with gold and cecropin-mellitin antimicrobial hybrid peptide as AMP test samples.

Different surfaces used for direct contact assay with HUVEC and NDHF cells. In addition, samples of gold and AMP coated surfaces without any cell culture were also observed for control reasons.

Scanning electron microscope imaging was performed using a FEG ESEM/EDS/EBSD FEI Quanta 400 FEG ESEM / EDAX Genesis X4M (need to write just model name).

For quantification purposes ten images of different fields of the different samples in a total of three replicates (for NDHF cells only a number of two replicates was imaged) per condition was performed. The number of cells present per area of surface was calculated and plotted in a graphic to better understand the total number of cells present in each surface type. Images for this purpose were captured using a 200x magnification. Furthermore, cell morphology was detailed analyzed using images captured at higher magnifications such as 5000x, 40000x, 100000x or 200000x.

More accurate interpretation of cells and particles present in these samples was performed using Energy Dispersive Spectrum (EDS), which was acquired for specific area spots and to allow proper differentiation between organic and gold material.

### Lactate Dehydrogenase (LDH) Release Based Assay

LDH release assay was performed for cells tretaed with different surfaces in similar condition used for direct contact measurements. Three replicates was used per condition to get statstically significant data .The lactate dehydrogenase assay is simple and accurate method and the assay is based on the reduction of NAD to NADH by LDH. It allows a determination of the membrane integrity as a function of the amount of cytoplasmic LDH released into the media.

LDH release and respective total LDH measurements were determined according to manufacturer's procedure (preferably, Sigma, Cat. Number TOX7). For total LDH quantification, LDH assay lysis buffer was used prior to recovering the sample for testing. For all samples, a volume of LDH assay mixture, equal to twice the volume of media to be tested was added in opaque plate having media from cells treated with different surfaces. The plate was protected from light with aluminum foil and incubated for 20-30 minutes. Using a spectrophotometer (preferably, Synergy Mx, Biotek Instruments), the absorbance was determined at a wavelength of 490 nm and background at 690 nm. Later value was subtracted to the primary wavelength obtained reading (490 nm).

### Membrane Potential Assessment

Cells were seeded in 8-well sterile Labtek chambers and cultured for at least 24h. Subsequently, attached cells were stained after being washed with a solution of 0.5% of bovine serum albumin in phosphate buffer saline. A mixture of propidium iodide and 3,3'-dipentyloxacarbocyanine iodide (DiOC5) (1 mg/mL) was allowed to react with the cells for 10 min at 37 °C. The tretaed cells were then washed with PBS and were imaged using a fluorescent microscope (Zeiss, Germany). At least three images of each condition were captured for quantification purposes. Pictures were analyzed using Image J 1.45S software (National Institutes of Health, USA) and both channels were examined separately. The image of the channel corresponding to the propidium iodide dye that labels dead cells was inverted using the mentioned software prior to counting. Furthermore, the images of the channel corresponding to DiOC 5 were used as acquired and cell counter pluggin utilized for manual quantification of total number of cells expressing the molecule of interest (not clear). All the data was then plotted accordingly.

### Statistical Analysis

For all the experiments at least three replicates were analyzed. Data was analyzed using Prism Software for Macintosh (Version 4.0b, GraphPad Software, Inc., 2004) and plotted in the graphic format. Statistical t-student test was performed to compare different groups using a confidence interval of 95%.

### RESULTS

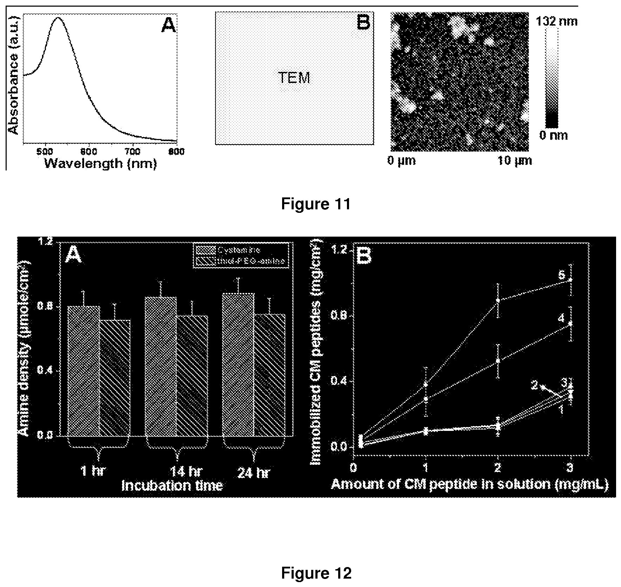

Gold nanoparticles (Au NPs) were synthesized using well-known Turkevich method (Wenzel, R. N. Ind. Eng. Chem. 1936, 28, 988). Citrate stabilized gold nanoparticles were characterized using UV-Vis spectroscopy and TEM analysis. Figure 1A shows the UV-vis spectrum of gold nanoparticles with surface plasmon resonance (SPR) band at 520 nm, indicating the synthesis of spherical gold nanoparticles. Supporting evidence of this observation was obtained by TEM analysis of this solution (Figure 11). TEM image shows the synthesis of spherical gold nanoparticles with the size of 20 nm (Figure 1B). The synthesized gold nanoparticles were used for the fabrication of gold films on the glass slide. AFM image shows uniform distribution of gold nanoparticles on silane functionalized glass slide (Figure 1C). Amine molecules are often employed to functionalize surfaces to provide compatibility to biomolecules as amine containing polymers are known to interact strongly via physisorption on glass surfaces (Song, J.; Chen, J.; Klapperich, C. M.; Eng, V.; Bertozzi, C. R. J. Mater. Chem. 2004, 14, 2643). Silane group of 3-amino propyltrymethoxysilane reacts with the glass surface via silaxone linkage while the amine groups are available to interact with the gold nanoparticles via electrostatic interactions (Karakouz, T.; Maoz, B.M.; Lando, G.; Vaskevich, A.; Rubinstein, I. ACS Appl. Mater. Interfaces 2011, 3, 978).

In order to immobilize CM peptide on the Au NPs coated surfaces, cystamine and thiol-PEG-amine were chosen as spacers to functionalize surfaces (scheme 1). Cystamine reacts with gold on cleavage of sulfer-sulfer disulfide bond and thiol-PEG amine reacts via the thiol group, leading to availability of two amine groups per molecules on the cystamine coated surface compared to one amine group per molecule on thiol-PEG-amine coated surface (Rai, A.; Perry, C. C. Langmuir 2010, 26, 4152). However, picrate test shows 1.5 time higher amine groups on cystamine than thiol-PEG-amine coated surfaces after 24 hr incubation, which is in good agreement with XPS analysis (Figure 3 and Table 2 and 3):

**Table 2 - Amine density and contact angle measurements of as prepared films on Au NPs coated surfaces.**

| Surface → | AuNPs surface | AuNPs-cystamine surface | CS surface | CM-CS surface | AuNPs-thiol-PEG-amine surface | NS surface | CM-NS surface |
|---|---|---|---|---|---|---|---|
| Amine density (µmole/cm²) after 24 hr | 0 | 0.879 ± 0.09 | 0 | | 0.754 ± 0.098 | 0 | |
| Contact angle (°) | 35 ± 2 | 52 ± 1 | | 70 ± 2 | 30 ± 3 | | 75 ± 2 |
| Surface → | | N:C 1:1 | N:C 2:1 | N:C 3:1 | N:C 4:1 | N:C 5:1 | |
| Contact angle (°) | | | | | | | |

**Table 3: Atomic composition (At %) of surfaces at various coating steps measured**

| | | | | |
|---|---|---|---|---|
| | C1s | N1s | O1s | S2p |
| Au NPs | 5.64 | 0.27 | 50.99 | 0 |
| AuNPs-cystamine surface | 6.72 | 0.96 | 53.70 | 0 |
| CS surface | 11.32 | 1.33 | 54.96 | 0 |
| CM-CS surface | 23.63 | 4.47 | 45.26 | 0.29 |
| AuNPs-thiol-PEG-amine surface | 17.46 | 0.81 | 50.72 | 0 |
| NS surface | 20.64 | 0.99 | 55.07 | 0 |
| CM-NS surface | 28.47 | 4.7 | 40.38 | 0.48 |

There is a small increase in the number of amine groups on both surfaces after 1 hr of incubation; however it is crucial to incubate for 24 hr to obtain the organized monolayer on the surface (Figure 3) ((a) Wang, H.; Chen, S. F.; Li, L. Y.; Jiang, S. Y. Langmuir 2005, 21, 2633. b) Dietrich, P. M.; Graf, N.; Gross, T.; Lippitz, A.; Krakert, S.; Schupbach, B.; Terfort, A.; Unger, W. E. S. Surf. Interface Anal. 2010, 42, 1184).

Sulfo-GMBS is heterobifunctional cross-linker that contains NHS ester and meleimide groups. After modification with sulfo-GMBS, the amine band at 1630 cm-1 disappeared due to covalent reaction with N-hydroxysuccinimide (NHS) ester group of sulfo-GMBS and new bands appears. Similarly, increase in the number of nitrogen (N%) oxygen (%O) was observed in XPS analysis for the sulfo-GMBS modified surfaces (Table 3). No amine groups were detected using picrate test, indicating the modification of amine groups with sulfo-GMBS (Table 2). CM peptide having sulfhydryl group (-SH group) at C-terminus reacts covalently with freely available maleimide group of sulfo-GMBS on the Au NPs coated surfaces. Curves in Figure show the characteristic amide-I (C=O stretching mode) and amide-II (N-H bending mode at 1640 and 1560 cm-1 respectively. XPS analysis shows the presence of sulfur element on only CM peptide coated surfaces than other surfaces, indicating the presence of covalently attached CM peptide on both NS and CS surfaces (Table 3).

The amount of covalently immobilized CM peptide on the NS surface is estimated to be higher than that on the CS surface although the number of amine group is higher on cystamine functionalized surface. XPS analysis shows that % C, N and O are higher on the NS surface (Table 3). This is probably due to the fact that flexible and long chain thiol-PEG-amine on the NS surface promotes more immobilization, lateral diffusion and dense packing by interfacial rearrangement of CM peptide compared to the stiffed CS surface where steric hindrances between CM peptides dominate to inhibit the further adsorption (a) Hylton, D. M.; Klee, D.; Fabry, M.; Hocker, J. Colloid Interface Sci. 1999, 220, 198. b) Kleijn, M.; Norde, W. Heterog. Chem. Rev. 1995, 2, 157). Moreover, the coating of gold nanoparticles on the glass surface generates a high roughness, which in turn leads to immobilization of a large amount of peptide per cm2 surface area than other reported procedures ((b) Gao, G.; Yu, K.; Kindrachuk, J.; Brooks, D. E.; Hancock, R. E.W.; Kizhakkedathu, J. N. Biomacromol. 2011, 12, 3715. c) Gabriel, M.; Nazmi, K.; Veerman, E. C.; Amerongen, A. V. N.; Zentner, A. Bioconj.Chem. 2006, 17, 548 and Gao, G.; Lange, D.; Hilpert, K.; Kindrachuk, J.; Zou, Y.; Cheng, J. T. J.; Kazemzadeh-Narbat, M.; Yu, K.; Wang, R.; Straus, S. K.; Brooks, D. E.; Chew, B. H.; Hancock, R. E.W.; Kizhakkedathu, J. N. Biomater. 2011, 32, 3899 and Kazemzadeh-Narbat, M.; Kindrachuk, J.; Duan, K.; Jenssen, H.; Hancock, R. E. W.; Wang, R. Biomater. 2010, 31, 9519). Figure 3B shows a large amount of immobilization of CM peptide on both surfaces with increasing amount, however a little adsorption is found on the gold, cystamine and thiol-PEG-amine coated surfaces without being modified with sulfo-GMBS due to nonspecific physical interactions. Chemical composition of films changed at every stage of coatings process, resulting into the variation of contact angle (Table 2). The trend in increase of contact angle reflects the varying wettability of different functional groups present on both surfaces, which are in commensurate with XPS analysis (Table 2 and 3). Au NPs and amine-modified surfaces are hydrophilic while the CM peptide coated surface is hydrophobic due to presence of a large percentage of hydrophobic amino acids, which contributes in generation of hydrophobic behavior to the peptide coated surfaces (Table 2).

CM peptide is highly cationic in nature and adopts well defined structures upon interactions with bacterial membranes in solution and these structural changes are related to their potent antimicrobial activity ((a) Zasloff, M. Nature 2002, 415, 389. b) Brogden, K.A. Nat. Rev. Microbiol. 2005, 3, 238. c) Bastos, M.; Bai, G.; Gomes, P.; Andreu, D.; Goormaghtigh, E.; Prieto, M. Biophys J. 2008, 94, 2128. d) Melo, M. N.; Ferre, R.; Castanho, M. A. Nat. Rev. Microbiol. 2009, 7, 245). In case of tethered CM peptide on the surface, autolysis and bacterial death could be triggered due to the disturbance of surface electrostatics of bacterial membrane or the penetration of peptide into bacterial membrane upon electrostatic interactions with local positively charged surface ((d) Hilpert, K.; Elliott, M.; Jenssen, H.; Kindrachuk, J.; Fjell, C. D.; Korner, J.; Winkler, D. F. H.; Weaver, L. L.; Henklein, P.; Ulrich, A. S.; Chiang, S. H. Y.; Farmer, S. W.; Pante, N.; Volkmer, R.; Hancock, R. E. W. Chem. Biol. 2009, 16, 58 and Jelokhani-Niaraki, M.; Prenner, E. J.; Kay, C. M.; McElhaney, R. N.; Hodges, R. S. J. Pept. Res. 2002, 60, 23). Thus, high charge density on the surface associated with the amount of peptide, flexibility and length of spacer may contribute to antimicrobial activity of CM peptide tethered surfaces. In order to verify the activity of CM peptide tethered surfaces, different amounts of CM peptide immobilized surfaces were tested against Gram-positive (S. aureus) and Gram-negative (E. coli) bacteria. As incubation time increases, CFU of S. aureus and E. coli decreases with increasing amount of CM peptide on the NS and the CS surfaces, showing evidence for the effectiveness of the CM peptide tethered surfaces for their antibacterial activity (Figure 4). In case of CM peptide tethered CS surface, 98% bacteria (E. coli and S. aureus) were killed after 4 h of incubation although the surface with a low amount of immobilized CM peptide (0.29 mg/cm2) was unavailable to kill bacteria after 2 h incubation (Figure 4A and C). In general, the CS surface with a low amount of peptide acts as bacteriostatic while the CS surface with a large amount of peptide acts as bactericidal as also observed by others (Humblot, V.; Yala, J-F.; Thebault, P.; Boukerma, K.; Hequet, A.; Berjeaud, J-M.; Pradier, C-M. Biomater. 2009, 30, 3503). In contrast to CS surface, different amounts of CM peptide tethered on the NS surfaces act as bacteriostatic with 98% bacteria being killed by surface having 0.89 and 0.38 mg/cm2 of peptide after 2 and 4 h of incubation respectively (Figure 4B and D). Interestingly, the NS surface containing 1.02 mg/cm2 of peptide kills 100% bacteria after 1.30 hr of incubation, which makes this surface ideal for further study (Figure 4B and D).

The control surfaces such as cystamine and thiol-PEG-amine coated gold surfaces did not show any antimicrobial activity (data not shown). The enhanced antimicrobial activity of CM peptide on the NS surface is due to combined effect of large amount of peptide, flexibility and long chain of thiol-PEG-amine molecule. The long chain and swelling of thiol-PEG-amine in the presence of buffer due to its water swelling property moves the CM peptide further from the surface thereby reducing the interaction between the peptide and the surface with maintenance of CM peptide activity ((a) Banerjee, I.; Pangule, R. C.; Kane, R. S. Adv. Mater. 2011, 23, 690. b) Liu, Z.; Wang, L.; Bao, C.; Li, X.; Cao, L. Dai, K.; Zhu, L. Biomacromol. 2011, 12, 2389). Moreover, the flexibility of thiol-PEG-amine molecule provides lateral mobility to peptide and a large amount of peptide on the surface restricts conformational freedom of CM peptide, resulting into better interaction with bacterial membrane and thus the enhancement of bactericidal activity (Onaizi, S. A.; Leong, S. S. J. Biotechnol. Adv. 2011, 29, 67. b) Costa, F. Carvalho, I. F. Montelaro, R. C. Gomes, P.; Martins, C. L. Acta Biomaterialia 2011, 7, 1431 and a) Humblot, V.; Yala, J-F.; Thebault, P.; Boukerma, K.; Hequet, A.; Berjeaud, J-M.; Pradier, C-M. Biomater. 2009, 30, 3503. b) Gao, G.; Yu, K.; Kindrachuk, J.; Brooks, D. E.; Hancock, R. E.W.; Kizhakkedathu, J. N. Biomacromol. 2011, 12, 3715. c) Gabriel, M.; Nazmi, K.; Veerman, E. C.; Amerongen, A. V. N.; Zentner, A. Bioconj.Chem. 2006, 17, 548. d) Hilpert, K.; Elliott, M.; Jenssen, H.; Kindrachuk, J.; Fjell, C. D.; Korner, J.; Winkler, D. F. H.; Weaver, L. L.; Henklein, P.; Ulrich, A. S.; Chiang, S. H. Y.; Farmer, S. W.; Pante, N.; Volkmer, R.; Hancock, R. E. W. Chem. Biol. 2009, 16, 58. e) Steven, H.D.; Hotchkiss, J. H. J. Appl. Polym. Sci. 2008, 110, 2665. f) Appendini, P.; Hotchkiss, J. H. J. Appl. Polym. Sci. 2001, 81, 609. g) Cho, W. M.; Joshi, B. P.; Cho, H.; Lee, K. H. Bioorg. Med. Chem. Lett. 2007, 17, 5772.). These results suggest that a low amount of peptide along with a short spacer chain length promotes bacteriostatic property perhaps by disturbing a low level of bacterial membrane electrostatics. While a high amount of peptide with short or long spacer chain length induces bactericidal activity in combination with penetration of peptide in the membrane and disturbance of membrane electrostatics (Figure 4).

The mechanism of antimicrobial activity is supported by AFM analysis. A height mode AFM image shows severely damaged and punctured membrane of E. coli treated with CM peptide tethered NS surface, while the smooth cell wall of E. coli is observed in control experiment where bacteria is incubated in Au NPs coated surface (Figure 5A and C). It may be possible that the electrostatic disturbance by tethered peptide on NS surface creates pores in bacterial cell wall followed by penetration of the peptide to form "hole", which lead to leakage of cell contents and death of bacteria (Figure 5A and D). The "hole" in AFM image is observed due to free movement of the damaged membrane compared to intact membrane by the AFM tip during scanning (4a). In phase mode image, it is clearly visible that the outer cell wall layer is intact after exposure to the CM peptide tethered NS surface (Figure 5D). E. coli treated with CM peptide tethered CS surface (0.29 mg/cm2) has roughened cell wall without any "holes" due to minor disturbance of electrostatics of membrane (Figure 5B and E), which corroborates with the observed bacterostatic property of the surface (Figure 4A). The cross sectional analysis revealed the increased roughness with decreased height due to collapsed cell wall of E. coli for CM peptide tether NS surface than that of less rough cell wall of E. coli for CM peptide tethered CS surface (Figure 5G-I). Thus, our results strongly suggest that the suitable surface chemistry and the amount of antimicrobial peptide tethered on the surface are critical in obtaining the potent antimicrobial activity.

The orientation of CM peptide immobilized on the surface is an important criteria to achieve its maximum activity. Figure 6A shows the antimicrobial activity of CM peptide randomly immobilized on thiol-PEG-acid modified surface. The comparably low killing (48 %) of E. coli was found after 4 h of incubation, which is likely related to the fact that the most of basic amino acids might not be accessible to bacterial membrane (Figure 6A). On the other hand, the C-terminal conjugation governs appropriate orientation of CM peptide along with free basic amino acids being present at the N-terminal and facilitates better interaction with negatively charged bacterial membrane, which could explain deleterious effect of tethered CM peptide (98% activity) (Figure 4). Other researchers have also observed similar trends although there is ambiguity in the literature about the effect of orientation on the activity of antimicrobial peptide (Haynie, S.L.; Crum, G. A.; Doele, B. A. Antimicrob. Agents Chemotherap. 1995, 39, 301 and b) Gao, G.; Yu, K.; Kindrachuk, J.; Brooks, D. E.; Hancock, R. E.W.; Kizhakkedathu, J. N. Biomacromol. 2011, 12, 3715. c) Gabriel, M.; Nazmi, K.; Veerman, E. C.; Amerongen, A. V. N.; Zentner, A. Bioconj.Chem. 2006, 17, 548.)

To the best of our knowledge, there is no research paper available related to antimicrobial activity of the tethered peptide in the presence of human serum. The CM peptide tethered NS surface did not show any antimicrobial activity after the surface being incubated in 20% human serum for 2 h. It is possible that human serum adsorbs on the top of CM peptide tethered surface and inhibits the activity of CM peptide. Thus, the coating procedure was slightly modified by introducing different molar ratios of thiol-PEG-amine to thiol-PEG-acid molecules. The rationale behind using mixed PEG molecules was to create the zwitterionic type surface and the high density of PEG molecules on the surface to prevent the adsorption of human serum ((a) Banerjee, I.; Pangule, R. C.; Kane, R. S. Adv. Mater. 2011, 23, 690., and a) Holmlin, R. E.; Chen, X.; Chapman, R. G.; Takayama, S.; Whitesides, G. M. Langmuir 2001, 17, 2841. b) Vaisocherova, H.; Yang, W.; Zhang, Z.; Cao, Z.; Cheng, G.; Pillarik, M.; Homola, J.; Jiang, S. Anal. Chem. 2008, 80, 7894. c) Chapman, R. G.; Ostuni, E.; Yan, L.; Whitesides, G. M. Langmuir 2000, 16, 6927). Figure 6B shows that 1:1 ratio of N:C surface is capable to kill 85 % population of E. coli compared to other molar ratio surfaces after 4 h of incubation. Contact angle measurement indicates that the 1:1 ratio surface is more hydrophilic than other molar ratios surfaces, which renders this surface resistant to serum adsorption, thereby protecting the antimicrobial activity of tethered CM peptide (Table 2). In line with the above procedure, the CM peptide tethered on the Au NPs coated titanium surface showed comparably similar antimicrobial activity in 4 h of incubation in the presence of human serum (Figure 6C). The result suggests that this procedure might be useful to coat the antimicrobial peptide on orthopaedic and dental implants for their biomedical applications. The coating of CM peptide on Au NPs coated Ti surface was very robust with antimicrobial activity being maintained after five cycles of repeated use (Figure 6D). The five cycles were completed in 10 days with frequency of 2 days for each cycle. The antimicrobial activity was slightly reduced after each cycle of use but efficiently killed the bacteria (90%) even after five cycles relative to bacteria incubated with control Ti surface (Figure 6D).

### Cell Cytotoxicity Analysis

Cecropin-mellitin (CM) is one antimicrobial peptide in use for many years.

Different concentrations of soluble cecropin-mellitin peptide were placed in contact with both NDHF and HUVEC cells previously cultured for 24 h in TCPS 24-well plates. The objective was to prove that CM peptide does not affect cells while being active killing bacteria cells.

In these experiments it was detected that high levels of soluble CM peptide had statistical significant prejudicial effects in NDHF cell metabolic activity after 24 h of direct contact between cells and soluble peptide. It was found that CM concentrations between 10 and 2.5 µg/mL were particularly effective decreasing cell metabolic activity measured indirectly through the ability of cells to transform MTT salt in formazan crystals. Similar results were obtained for experiments with HUVEC. For this cell type negative influence of soluble CM peptide was also detected for the concentration of 1 µg/mL, which confirms that endothelial cells are a cell system not robust as fibroblasts or this is supported by the higher value of standard deviation determined for the 2.5 µg/mL CM soluble peptide condition.

Extraction assays were also performed using, for that purpose, media that has been in contact with different surfaces for 24h under gentle agitation (150 RPM) at 37°C. Here, the main objective was to evaluate if there were some particles that would be leaching out from the surfaces and would therefore affect the behavior of cells that were in culture. It was detected that, in the case of HUVEC cell cultures, extracts coming from AMP modified surfaces were drastically affected cells and their metabolic activity decreased. A negative control was added to this set of conditions in order to destroy the cells and have a negative value for this influence, in this case hydrogen peroxide, which was effective for this purpose.

Direct contact of HUVEC cells with modified surfaces is shown below. Results show that there are differences between samples modified with AMP peptides and the ones modified with PEG-amine (PEG-NH2) and PEG-COOH only (NC chemistry samples). Controls for this experiment comprised cells seeded in tissue culture treated plastic (TCPS) and in non-modified glass coverslips that were previously washed in ethanol and dried with nitrogen gas as well as sterilized as all the others surfaces.

Significative differences between AMP modified surfaces and unmodified, either gold treated and gold treated plus NC chemistry, were found. Therefore it seems probable that the presence of the peptide is influencing the cell behaviour in contact with these specific surfaces.

Further experiments were also performed with normal human dermal fibroblasts (NDHF) in order to confirm if the same results would be obtained. Results here obtained showed that fibroblasts in direct contact with AMP modified surfaces had the metabolic activity decreased. Consequently, similar results were acquired for both cell types.

Fibroblasts and human umbilical vein endothelial cells would have different roles in the maintenance of specific cell populations in the adult human tissue and for this reason were here investigated. In this particular assay they behaved the same although endothelial cells are known to be more sensitive cells than fibroblasts.

### References

1. (a) De Simone, M.; Ghosh, P. S.; Rotello, V. M., <De_Applications of NPs in biology_Adv Mater 08.pdf>. Advanced Materials 2008, 20, 4225-4241; (b) Ghosh, S. K.; Pal, T., <Ghosh_Interparticle coupling effect on SPR of gold NPs_Chem Rev 07.pdf>. Chemical Review 2007, 107, 4797-4862; (c) Daniel, M. C.; Astruc, D., <Daniel_Gold NPs assembly supermol chem and applications_Chem Rev 04.pdf>. Chemical Review 2004, 104, 293-346.
2. (a) Rai, A.; Prabhune, A.; Perry, C. C., Antibiotic mediated synthesis of gold nanoparticles with potent antimicrobial activity and their application in antimicrobial coatings. Journal of Materials Chemistry 2010, 20 (32), 6789-6798; (b) Sambhy, V.; MacBride, M. M.; Peterson, B. R.; Sen, A., <AgBr nps antimicrobial activity JACS 06.pdf>. Journal of American Chemical Society 2006, 128, 9798-9808; (c) Gu, H.; Ho, P. L.; Tong, E.; Wang, L.; Xu, B., <Vancomycin coated Au nps for antibac NL 03.pdf>. Nano Letters 3 (9), 1261-1263; (d) Duran, N.; Marcato, P. D.; De Souza, G. I. H.; Alves, O. L.; Esposito, E., <Antibac effect of Ag nps produced by Fungus.pdf>. Journal of Biomedical Nanotechnology 2007, 3, 203-208; (e) Kim, J. S.; Kuk, E.; Yu, K. N.; Kim, J. H.; Park, S. J.; Lee, H. J.; Kim, S. H.; Park, Y. K.; Park, Y. H.; Hwang, C. Y.; Kim, Y. K.; Lee, Y. S.; Jeong, D. H.; Cho, M. H., Antimicrobial effects of silver nanoparticles. Nanomedicine 2007, 3 (1), 95-101.
3. (a) Yuan, W.; Jiang, G.; Che, J.; Qi, X.; Xu, R.; Chang, M. W.; Chen, Y.; Lim, S. Y.; Dai, J.; Chan-Park, M. B., <Dep of Ag nps on CNT and its antimicrobial effects JPC 08.pdf>. J Phys Chem C 2008, 112 (48), 18754-18759; (b) Nath, S.; Kaittanis, C.; Tinkham, A.; Perez, J. M., <Dextran coated Au nps for antimic susc Anal Chem 08.pdf>. Analytical Chemistry 2008, 80 (4), 1033-1038; (c) Saha, B.; Bhattacharya, J.; Mukherjee, A.; Ghosh, A.; Santra, C.; Dasgupta, A.; Karmakar, P., In Vitro Structural and Functional Evaluation of Gold Nanoparticles Conjugated Antibiotics. Nanoscale Research Letters 2007, 2 (12), 614 - 622; (d) Veerapandian, M.; Yun, K., Functionalization of biomolecules on nanoparticles: specialized for antibacterial applications. Applied Microbiology and Biotechnology 2011, 90 (5), 1655-1667; (e) Carmona, D.; Lalueza, P.; Balas, F.; Arruebo, M.; Santamaria, J., Mesoporous silica loaded with peracetic acid and silver nanoparticles as a dual- effect, highly efficient bactericidal agent. Microporous and Mesoporous Materials 2012, 161, 84-90; (f) Chen, S.; Guo, Y.; Chen, S.; Yu, H.; Ge, Z.; Zhang, X.; Zhang, P.; Tang, J., Facile preparation and synergistic antibacterial effect of three-component Cu/TiO2/CS nanoparticles. Journal of Materials Chemistry 2012, 22 (18), 9092-9099; (g) Strong bactericidal synergy between peracetic acid and silver-exchanged zeolites. Microporous and Mesoporous Materials 2012; (h) Parashar, U. K.; Kumar, V.; Bera, T.; Saxena, P. S.; Nath, G.; Srivastava, S. K.; Giri, R.; Srivastava, A., Study of mechanism of enhanced antibacterial activity by green synthesis of silver nanoparticles. Nanotechnology 2011, 22 (41), 415104; (i) Li, P.; Li, J.; Wu, C.; Wu, Q.; Li, J., <Li-Synergistic antibac.pdf>. Nanotechnology 2005, 16, 1912-1917; (j) Allahverdiyev Am Fau - Kon, K. V.; Kon Kv Fau - Abamor, E. S.; Abamor Es Fau - Bagirova, M.; Bagirova M Fau - Rafailovich, M.; Rafailovich, M., Coping with antibiotic resistance: combining nanoparticles with antibiotics and other antimicrobial agents. (1744-8336 (Electronic)).
4. (a) Batarseh, K. I., Anomaly and correlation of killing in the therapeutic properties of silver (I) chelation with glutamic and tartaric acids. J Antimicrob Chemother 2004, 54 (2), 546-8; (b) Bridges, K.; Kidson, A.; Lowbury, E. J. L.; Wilkins, M. D., Gentamicin-Resistant and Silver-Resistant Pseudomonas in a Burns Unit. Brit Med J 1979, 1 (6161), 446-449; (c) Gupta, A.; Matsui, K.; Lo, J. F.; Silver, S., Molecular basis for resistance to silver cations in Salmonella. Nat Med 1999, 5 (2), 183-8; (d) Gupta, A.; Maynes, M.; Silver, S., Effects of halides on plasmid-mediated silver resistance in Escherichia coli. Appl Environ Microbiol 1998, 64 (12), 5042-5; (e) Gupta, A.; Silver, S., Molecular genetics - Silver as a biocide: Will resistance become a problem? Nat Biotechnol 1998, 16 (10), 888-888; (f) Gupta K Fau - Singh, V. P.; Singh Vp Fau - Kurupati, R. K.; Kurupati Rk Fau - Mann, A.; Mann A Fau - Ganguli, M.; Ganguli M Fau - Gupta, Y. K.; Gupta Yk Fau - Singh, Y.; Singh Y Fau - Saleem, K.; Saleem K Fau - Pasha, S.; Pasha S Fau - Maiti, S.; Maiti, S., Nanoparticles of cationic chimeric peptide and sodium polyacrylate exhibit striking antinociception activity at lower dose. (1873-4995 (Electronic)); (g) Qiu, Z.; Yu, Y.; Chen, Z.; Jin, M.; Yang, D.; Zhao, Z.; Wang, J.; Shen, Z.; Wang, X.; Qian, D.; Huang, A.; Zhang, B.; Li, J. W., Nanoalumina promotes the horizontal transfer of multiresistance genes mediated by plasmids across genera. Proc Natl Acad Sci U S A 2012, 109 (13), 4944-9.
5. (a) Lu, W.; Senapati, D.; Wang, S.; Tovmachenko, O.; Singh, A. K.; Yu, H., <Effect of surface coating on toxicity of silver nps .pdf>. Chemical Physics Letters 2010, 487, 92-96; (b) Wang, S.; Lu, W.; Tovmachenko, O.; Rai, U. S.; Yu, H.; Ray, P. S., <Challenge in understanding size and shape dep toxicity of gold NPS.pdf>. Chemical Physics Letters 2008, 463, 145-149; (c) Oh, W.-K.; Kim, S.; Choi, M.; Kim, C.; Jeong, Y. S.; Cho, B. R.; Hahn, J.-S.; Jang, J., <Cellular uptake cytotoxicity and immune res of Si Ti hollow nps ACS nano 10.pdf>. ACS Nano 2010, 4 (9), 5301-5313; (d) Nel, A.; Xia, T.; Madler, L.; Li, N., <review_toxicity_nanomaterials_science05.pdf>. Science 2006, 311, 622-627; (e) AshaRani, P. V.; Low Kah Mun, G.; Hande, M. P.; Valiyaveettil, S., Cytotoxicity and genotoxicity of silver nanoparticles in human cells. ACS Nano 2009, 3 (2), 279-90; (f) Bakshi, M. S., <Nanoshpe cont tendency of PL and proteins, Protein and nps composites JPCC 11.pdf>. J Phys Chem C 2011, 115 (29), 13947-13960.
6. (a) Liu, L.; Xu, K.; Wang, H.; Tan, P. K. J.; Fan, W.; Venkatraman, S. S.; Li, L.; Yang, Y.-Y., <Liu_Self assembled cationic peptide nanoparticles_Nature Nanotech .pdf>. Nature Nanotechnology 2009, 4, 457-463; (b) Bi, L.; Yang, L.; Narsimhan, G.; Bhunia, A. K.; Yao, Y., Designing carbohydrate nanoparticles for prolonged efficacy of antimicrobial peptide. J Control Release 2011, 150 (2), 150-6; (c) Ruden, S.; Hilpert, K.; Berditsch, M.; Wadhwani, P.; Ulrich, A. S., Synergistic interaction between silver nanoparticles and membrane-permeabilizing antimicrobial peptides. Antimicrob Agents Chemother 2009, 53 (8), 3538-40.
7. (a) D., A.; Ubach, J.; Boman, A.; Wahlin, B.; Wade, D.; Merrifield, R. B.; Boman, H. G., <Shortned cecropin A melitting hybrid FEBS latter.pdf>. FEBS Letter 1992, 296, 190-194; (b) Merrifield, R. B.; Juvvadi, P.; Andreu, D.; Ubach, J.; Boman, A.; Boman, H. G., <Merrifield_Retro and retroenantio analogs of CM hybrids_ Proc Natl Acad Sci USA 1995.pdf>. Proc Natl Acad Sci U S A 1995, 92, 3449-3453; (c) Edlund, C.; Hedberg, M.; Engstrom, A.; Flock, J.-I.; Wade, D., <Antianaerobic act of cecropin melittin pep.pdf>. Clinical Microbiology and Infection 1998, 4 (4), 181-185; (d) Giacometti, A.; Cirioni, O.; Kamysz, W.; D'Amato, G.; Silvestri, C.; Del Prete, M. S.; 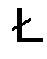 ukasiak, J.; Scalise, G., Comparative activities of cecropin A, melittin, and cecropin A-melittin peptide CA(1-7)M(2-9)NH2 against multidrug-resistant nosocomial isolates of Acinetobacter baumannii. Peptides 2003, 24 (9), 1315-1318; (e) Merrifield EI Fau - Mitchell, S. A.; Mitchell Sa Fau - Ubach, J.; Ubach J Fau - Boman, H. G.; Boman Hg Fau - Andreu, D.; Andreu D Fau - Merrifield, R. B.; Merrifield, R. B., D-enantiomers of 15-residue cecropin A-melittin hybrids. (0367-8377 (Print)).
8. (a) Lowe, P. J.; Temple, C. S., Calcitonin and Insulin in Isobutylcyanoacrylate Nanocapsules: Protection Against Proteases and Effect on Intestinal Absorption in Rats. Journal of Pharmacy and Pharmacology 1994, 46 (7), 547-552; (b) Eby, D. M.; Farrington, K. E.; Johnson, G. R., Synthesis of bioinorganic antimicrobial peptide nanoparticles with potential therapeutic properties. Biomacromolecules 2008, 9 (9), 2487-94.
9. (a) Diamanti, S.; Elsen, A.; Naik, R.; Vaia, R., Relative Functionality of Buffer and Peptide in Gold Nanoparticle Formation. J Phys Chem C 2009, 113 (23), 9993-9997; (b) Serizawa, T.; Hirai, Y.; Aizawa, M., Novel Synthetic Route to Peptide-Capped Gold Nanoparticles. Langmuir 2009, 25 (20), 12229-12234; (c) Stanley, S. K.; Becker, M. L.; Lin, E. K.; Wu, W. L., Inhibitory Effects of a Phage-Derived Peptide on Au Nanocrystal Nucleation and Growth. Langmuir 2009, 25 (18), 10886-10892; (d) Scari, G.; Porta, F.; Fascio, U.; Avvakumova, S.; Dal Santo, V.; De Simone, M.; Saviano, M.; Leone, M.; Del Gatto, A.; Pedone, C.; Zaccaro, L., Gold nanoparticles capped by a GC-containing peptide functionalized with an RGD motif for integrin targeting. Bioconjug Chem 2012, 23 (3), 340-9; (e) Tran, N. T.; Wang, T. H.; Lin, C. Y.; Tsai, Y. C.; Lai, C. H.; Tai, Y.; Yung, B. Y., Direct synthesis of Rev peptide-conjugated gold nanoparticles and their application in cancer therapeutics. Bioconjug Chem 2011, 22 (7), 1394-401.
10. (a) Dickerson, M. B.; Sandhage, K. H.; Naik, R. R., <Protein and peptide mediated syn of inog materials CHem Rev 0.pdf>. Chemical Review 2008, 108 (11), 4935-4978; (b) Slocik, J. M.; Stone, M. O.; Naik, R. R., Synthesis of gold nanoparticles using multifunctional peptides. Small 2005, 1 (11), 1048-52.
11. Kimling, J.; Maier, M.; Okenve, B.; Kotaidis, V.; Ballot, H.; Plech, A., <turkevich method for au nps syn JPCB06.pdf>. Journal of Physical Chemistry B 2006, 110 (32), 15700-15707.
12. Watzky, M. A.; Finke, R. G., <Watzky_Transition metal nanocluser formation kinetics_JACS 97.pdf>. J. Am. Chem. Soc. 1997, 119, 10382-10400.
13. (a) Prasad, B. L. V.; Stoeva, S. I.; Sorensen, C. M.; Klabunde, K. J., Digestive Ripening of Thiolated Gold Nanoparticles:,Äâ The Effect of Alkyl Chain Length. Langmuir 2002, 18 (20), 7515-7520; (b) Stoeva, S.; Klabunde, K. J.; Sorensen, C. M.; Dragieva, I., Gram-Scale Synthesis of Monodisperse Gold Colloids by the Solvated Metal Atom Dispersion Method and Digestive Ripening and Their Organization into Two- and Three-Dimensional Structures. Journal of the American Chemical Society 2002, 124 (10), 2305-2311; (c) Zheng, N.; Fan, J.; Stucky, G. D., One-Step One-Phase Synthesis of Monodisperse Noble-Metallic Nanoparticles and Their Colloidal Crystals. Journal of the American Chemical Society 2006, 128 (20), 6550-6551.
14. Xie, J.; Lee, J. Y.; Wang, D. I. C., <Xie_Seedless surfactantless syn of branched gold nanocrystals_Chem Mater 07.pdf>. Chemistry Materials 2007, 19, 2823-2830.
15. (a) Banfield, J. F.; Welch, S. A.; Zhang, H.; Ebert, T. T.; Penn, R. L., Aggregation-Based Crystal Growth and Microstructure Development in Natural Iron Oxyhydroxide Biomineralization Products. Science 2000, 289 (5480), 751-754; (b) Penn, R. L.; Banfield, J. F., Imperfect Oriented Attachment: Dislocation Generation in Defect-Free Nanocrystals. Science 1998, 281 (5379), 969-971; (c) Taubert, A.; Wiesler, U.-M.; Müllen, K., Dendrimer-controlled one-pot synthesis of gold nanoparticles with a bimodal size distribution and their self-assembly in the solid state. Journal of Materials Chemistry 2003, 13 (5), 1090-1093.
16. Prasad, B. L. V.; Stoeva, S. I.; Sorensen, C. M.; Klabunde, K. J., <Prasad_ Digestive ripening agent for gold NPs_Chem Mater03.pdf>. Chemistry Materials 2003, 15, 935-942.
17. Shimojo, K.; Niide, T.; Taguchi, T.; Naganawa, H.; Kamiya, N.; Goto, M., Facile, rapid and efficient biofabrication of gold nanoparticles decorated with functional proteins. Analyst 2012, 137 (10), 2300-3.
18. Haiss, W.; Thanh, N. T. K.; Aveyard, J.; Fernig, D., <Determination of concentration and size of gold nps from UV spectra Anal Chem 2007_David fernig.pdf>. Analytical Chemistry 2007, 79, 4215-4221.
19. Palma, R. D.; Peeters, S.; Van Bael, M. J.; Rul, H. V.; Bonroy, K.; Laureyn, W.; Mullens, J.; Borghs, G.; Maes, G., <Palma_Silane ligand exchange to make hydrophobic supermag nps_TGA for ligand calculation.pdf>. Chemistry Materials 2007, 19, 1821-1831.
20. Abrunhosa, F.; Faria, S.; Gomes, P.; Tomaz, I.; Pessoa, J. C.; Andreu, D.; Bastos, M., <Abrunhosa_Interaction and lipid conf of cecropin melletin peptide_JPCB 05.pdf>. Journal of Physical Chemistry B 2005, 109, 17311-17319.
21. Wade, D.; Boman, A.; Wahlin, B.; Drain, C. M.; Andreu, D.; Boman, H. G.; Merrifield, R. B., All-D amino acid-containing channel-forming antibiotic peptides. Proc Natl Acad Sci U S A 1990, 87 (12), 4761-5.
22. (a) Wiradharma, N.; Khoe, U.; Hauser, C. A.; Seow, S. V.; Zhang, S.; Yang, Y. Y., Synthetic cationic amphiphilic alpha-helical peptides as antimicrobial agents. Biomaterials 2011, 32 (8), 2204-12; (b) Sato, H.; Feix, J. B., Peptide-membrane interactions and mechanisms of membrane destruction by amphipathic alpha-helical antimicrobial peptides. Biochim Biophys Acta 2006, 1758 (9), 1245-56.
23. (a) Liu, Y.; Shipton, M. K.; Ryan, J.; Kaufman, E. D.; Franzen, S., <Liu_synthesis stability and internalization of gold NPs_Anal Chem 07.pdf>. Analytical Chemistry 2007, 79, 2221-2229; (b) Dominguez-Medina, S.; McDonough, S.; Swanglap, P.; Landes, C. F.; Link, S., In Situ Measurement of Bovine Serum Albumin Interaction with Gold Nanospheres. Langmuir: the ACS journal of surfaces and colloids 2012.
24. Ferre, R.; Badosa, E.; Feliu, L.; Planas, M.; Montesinos, E.; Bardaji, E., Inhibition of plant-pathogenic bacteria by short synthetic cecropin A-melittin hybrid peptides. Appl Environ Microbiol 2006, 72 (5), 3302-8.

## Claims

1. An antimicrobial coating composition for coating articles comprising:
a cecropin-mellitin peptide wherein said cecropin-mellitin peptide has a cysteine at C-terminal;
a nanoparticle bound to said cecropin-mellitin peptide wherein said nanoparticle is a metal nanoparticle or a nanoparticle covered by a metal layer, and wherein said metal is gold, titanium, or mixtures thereof;
a thiol-PEG-amine;
wherein the nanoparticle is functionalized by cysteine and thiol-PEG-amine and
wherein the composition is suitable to be bonded to the surface of the article.

2. The composition according to the previous claim further comprising a binder.

3. The composition according to the previous claim wherein said binder is a polymer.

4. The composition according to the previous claim wherein said polymer is a polycation or polyanion.

5. The composition according to claim 4 wherein said polymer is a polydopamine.

6. The composition according to any of the previous claims wherein said composition is bonded to the surface of the article by heating.

7. The composition according to any of the previous claims wherein said nanoparticles are obtainable using a cecropin-mellitin peptide as template.

8. The composition according to any of the previous claims further comprising a buffer.

9. The composition according to the previous claim wherein the nanoparticle is gold and the buffer is HEPES.

10. The composition according to the previous claims wherein the cecropin-mellitin peptide concentration is between 0.10 and 1 mM, preferentially between 0.1 and 0.5 mM.

11. The composition according to any of the previous claims wherein said nanoparticle has a size between 10-100 nm, preferably 15-50 nm, more preferably 20-30 nm.

12. The composition according to any of the previous claims for use in medicine, preferably as an anti-microbiological agent.

13. An article coated with the antimicrobial coating composition described in the claims 1 -12.

14. The article according to the previous claim wherein said article is a medical device, in particular wherein said medical device is an implant, a tongue depressor, a medical thermometer, a blood sugar meter, a medical robot, a microchip implant, a surgical tool or a neuroprosthesis.

## Patentansprüche

1. Eine antimikrobielle Beschichtungszusammensetzung zum Beschichten von Gegenständen, umfassend:
ein Cecropin-Melittin-Peptid, wobei das Cecropin-Melittin-Peptid ein Cystein am C-Terminus aufweist;
ein an das genannte Cecropin-Melittin-Peptid gebundener Nanopartikel, wobei der genannte Nanopartikel ein Metall-Nanopartikel oder ein mit einer Metallschicht beschichteter Nanopartikel ist, und wobei das genannte Metall Gold, Titan oder Mischungen davon ist;
ein Thiol-PEG-Amin;
wobei der Nanopartikel durch Cystein und Thiol-PEG-Amin funktionalisiert ist und
wobei die Zusammensetzung geeignet ist, an die Oberfläche des Gegenstandes gebunden zu werden.

2. Die Zusammensetzung nach dem vorangehenden Anspruch ferner umfassend ein Bindemittel.

3. Die Zusammensetzung nach dem vorangehenden Anspruch, wobei das genannte Bindemittel ein Polymer ist.

4. Die Zusammensetzung nach dem vorangehenden Anspruch, wobei das genannte Polymer ein Polykation oder Polyanion ist.

5. Die Zusammensetzung nach Anspruch 4, wobei das genannte Polymer ein Polydopamin ist.

6. Die Zusammensetzung nach einem der vorangehenden Ansprüche, wobei die genannte Zusammensetzung durch Erhitzen an die Oberfläche des Gegenstandes gebunden wird.

7. Die Zusammensetzung nach einem der vorangehenden Ansprüche, wobei die genannten Nanopartikel unter Verwendung eines Cecropin-Melittin-Peptids als Vorlage erhältlich sind.

8. Die Zusammensetzung nach einem der vorangehenden Ansprüche ferner umfassend einen Puffer.

9. Die Zusammensetzung nach dem vorangehenden Anspruch, wobei der Nanopartikel Gold und der Puffer HEPES ist.

10. Die Zusammensetzung nach den vorangehenden Ansprüchen, wobei die Cecropin-Melittin-Peptid-Konzentration zwischen 0,10 und 1 mM, bevorzugt zwischen 0,1 und 0,5 mM, liegt.

11. Die Zusammensetzung nach einem der vorangehenden Ansprüche, wobei der genannte Nanopartikel eine Größe zwischen 10-100 nm, bevorzugt 15-50 nm, besonders bevorzugt 20-30 nm aufweist.

12. Die Zusammensetzung nach einem der vorangehenden Ansprüche zur Verwendung in der Medizin, bevorzugt als antimikrobielles Mittel.

13. Ein Gegenstand, der mit der in den Ansprüchen 1-12 beschriebenen antimikrobiellen Beschichtungszusammensetzung beschichtet ist.

14. Der Gegenstand nach dem vorangehenden Anspruch, wobei der Gegenstand ein medizinisches Gerät ist, wobei das genannte medizinische Gerät insbesondere ein Implantat, eine Zungenspatel, ein medizinisches Thermometer, ein Blutzuckermessgerät, ein medizinischer Roboter, ein Mikrochip-Implantat, ein chirurgisches Instrument oder eine Neuroprothese ist.

## Revendications

1. Une composition de revêtement antimicrobien pour revêtir des articles comprenant :
un peptide de cécropine-mélittine dans laquelle ledit peptide de cécropine-mélittine a une cystéine au terminal C ;
une nanoparticule liée audit peptide de cécropine-mélittine dans laquelle ladite nanoparticule est une nanoparticule de métal ou une nanoparticule couverte d'une couche de métal, et dans laquelle ledit métal est de l'or, du titane, ou des mélanges de ceux-ci ;
une amine thiol-PEG;
dans laquelle la nanoparticule est fonctionnalisée par la cystéine et l'amine thiol-PEG et dans laquelle la composition est adaptée pour être liée à la surface de l'article.

2. La composition selon la revendication précédente comprenant également un liant.

3. La composition selon la revendication précédente dans laquelle ledit liant est un polymère.

4. La composition selon la revendication précédente dans laquelle ledit polymère est une polycation ou un polyanion.

5. La composition selon la revendication 4 dans laquelle ledit polymère est une polydopamine.

6. La composition selon l'une quelconque des revendications précédentes dans laquelle ladite composition est liée à la surface de l'article par chauffage.

7. La composition selon l'une quelconque des revendications précédentes dans laquelle lesdites nanoparticules peuvent être obtenues en utilisant un peptide de cécropine-mélittine en tant qu'exemple.

8. La composition selon l'une quelconque des revendications précédentes comprenant également un agent tampon.

9. La composition selon la revendication précédente dans laquelle la nanoparticule est de l'or et l'agent tampon est de l'HEPES.

10. La composition selon les revendications précédentes dans laquelle la concentration de peptide de cécropine-mélittine se situe entre 0.10 et 1 mM, préférablement entre 0.1 et 0.5 mM.

11. La composition selon l'une quelconque des revendications précédentes dans laquelle ladite nanoparticule a une taille située entre 10-100 nm, préférablement 15-50 nm, plus préférablement 20-30 nm.

12. La composition selon l'une quelconque des revendications précédentes pour une utilisation en médecine, préférablement en tant qu'agent anti-microbiologique.

13. Un article revêtu de la composition de revêtement antimicrobienne décrite dans les revendications 1-12.

14. L'article selon la revendication précédente dans lequel ledit article est un dispositif médical, en particulier dans lequel ledit dispositif médical est un implant, un abaisse-langue, un thermomètre médical, un glucomètre, un robot médical, un implant de micropuce, un instrument chirurgical ou une neuroprothèse.
